(19) Europäisches Patentamt / European Patent Office / Office européen des brevets

(11) **EP 4 408 262 B1**

(12) **EUROPEAN PATENT SPECIFICATION**

(45) Date of publication and mention
of the grant of the patent:
**25.03.2026 Bulletin 2026/13**

(21) Application number: **22854525.7**

(22) Date of filing: **06.12.2022**

(51) International Patent Classification (IPC):
**A61B 5/00** *(2006.01)* **A61B 5/0215** *(2006.01)*
**A61B 5/11** *(2006.01)*

(52) Cooperative Patent Classification (CPC):
**A61B 5/4839; A61B 5/0215; A61B 5/1107;
A61B 5/4875; A61B 5/686; A61B 5/7203;
A61B 5/7257; A61B 5/726; A61B 5/7267;**
A61B 2505/07; A61B 2505/09; A61B 2562/0233

(86) International application number:
**PCT/US2022/052039**

(87) International publication number:
**WO 2023/107508 (15.06.2023 Gazette 2023/24)**

(54) **LAP SIGNAL PROCESSING TO AUTOMATICALLY CALCULATE A/V RATIO**

LAP-SIGNALVERARBEITUNG ZUR AUTOMATISCHEN BERECHNUNG DES A/V-VERHÄLTNISSES

TRAITEMENT DE SIGNAL DE PRESSION AURICULAIRE GAUCHE (LAP) POUR CALCULER AUTOMATIQUEMENT UN RAPPORT A/V

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **07.12.2021 US 202163287003 P**
**07.12.2021 US 202163287009 P**
**07.12.2021 US 202163287016 P**
**17.12.2021 US 202163291253 P**
**17.12.2021 US 202163291270 P**

(43) Date of publication of application:
**07.08.2024 Bulletin 2024/32**

(73) Proprietor: **Edwards Lifesciences Corporation
Irvine, CA 92614-5688 (US)**

(72) Inventor: **KEIDAR, Yaron
3079892 Caesarea (IL)**

(74) Representative: **Venner, Julia Ann et al
Kilburn & Strode LLP
Lacon London
84 Theobalds Road
London WC1X 8NL (GB)**

(56) References cited:
• BRACKBILL T A ET AL: "Vectorcardiographic comparison of left ventricular hypertrophy in idiopathic hypertrophic subaortic stenosis, aortic stenosis, and aortic regurgitation", AMERICAN HEART JOURNAL, ELSEVIER, AMSTERDAM, NL, vol. 88, no. 3, 1 September 1974 (1974-09-01), pages 269 - 276, XP022946050, ISSN: 0002-8703, [retrieved on 19740901], DOI: 10.1016/0002-8703(74)90458-X
• ROSS J ET AL: "Clinical and hemodynamic observations in pure mitral insufficiency^*", AMERICAN JOURNAL OF CARDIOLOGY, CAHNERS PUBLISHING CO., NEWTON, MA, US, vol. 2, no. 1, 1 July 1958 (1958-07-01), pages 11 - 23, XP023194737, ISSN: 0002-9149, [retrieved on 19580701], DOI: 10.1016/0002-9149(58)90244-3
• TICE F D ET AL: "Transesophageal echocardiographic assessment of reversal of systolic pulmonary venous flow in mitral stenosis", AMERICAN JOURNAL OF CARDIOLOGY, CAHNERS PUBLISHING CO., NEWTON, MA, US, vol. 75, no. 1, 1 January 1995 (1995-01-01), pages 58 - 60, XP027424447, ISSN: 0002-9149, [retrieved on 19950101], DOI: 10.1016/S0002-9149(99)80528-1

EP 4 408 262 B1

- O'ROURKE R A ET AL: "Mitral valve regurgitation", CURRENT PROBLEMS IN CARDIOLOGY, ELSEVIER, AMSTERDAM, NL, vol. 9, no. 2, 1 May 1984 (1984-05-01), pages 1 - 52, XP023061691, ISSN: 0146-2806, [retrieved on 19840501], DOI: 10.1016/0146-2806(84)90021-5
- NEEMA ET AL: "Left Atrial Pressure Waveform: Does It Show Mitral Insufficiency?", JOURNAL OF CARDIOTHORACIC AND VASCULAR ANESTHESIA, ELSEVIER, AMSTERDAM, NL, vol. 22, no. 2, 28 March 2008 (2008-03-28), pages 318 - 320, XP022549749, ISSN: 1053-0770
- ABRAHAM WILLIAM T ET AL: "V-LAP Left Atrial Monitoring systEm for Patients with Chronic sysTOlic and Diastolic Congestive Heart FailuRe First-in-Human", JOURNAL OF CARDIAL FAILURE, CHURCHILL LIVINGSTONE, NAPERVILLE, IL, US, vol. 25, no. 8, 1 August 2019 (2019-08-01), XP085800389, ISSN: 1071-9164, DOI: 10.1016/ J.CARDFAIL.2019.07.211
- SNYDER R W ET AL: "Predictive value of prominent pulmonary arterial wedge V waves in assessing the presence and severity of mitral regurgitation", AMERICAN JOURNAL OF CARDIOLOGY, CAHNERS PUBLISHING CO., NEWTON, MA, US, vol. 73, no. 8, 15 March 1994 (1994-03-15), pages 568 - 570, XP023278754, ISSN: 0002-9149, [retrieved on 19940315], DOI: 10.1016/0002-9149(94)90335-2
- NITTER-HAUGE S ET AL: "Clinical and hemodynamic results after combined aortic and mitral valve replacement with the Lillehei-Kaster pivoting disc valve", AMERICAN HEART JOURNAL, ELSEVIER, AMSTERDAM, NL, vol. 97, no. 5, 1 May 1979 (1979-05-01), pages 599 - 607, XP022944747, ISSN: 0002-8703, [retrieved on 19790501], DOI: 10.1016/0002-8703(79)90187-X

**Description**

CROSS-REFERENCE TO RELATED APPLICATIONS

The current application claims the benefit of and priority under 35 U.S.C. § 119(e) to U.S. Provisional Patent Application No. 63/287,003 entitled "LAP SIGNAL PROCESSING TO AUTOMATICALLY CALCULATE A/V RATIO" filed December 7, 2021, to U.S. Provisional Patent Application No. 63/287,009 entitled "EXERTION RESPONSE DETECTION BY AN IMPLANTABLE SENSOR" filed December 7, 2021, U.S. Provisional Patent Application No. 63/287,016 entitled "IMPROVED SIGNAL PROCESSING OF BLOOD PRESSURE FROM AN IMPLANTABLE SENSOR BY PRE-CLASSIFICATION OF PATIENTS" filed December 7, 2021, U.S. Provisional Patent Application No. 63/291,253 entitled "SYSTEMS AND METHODS FOR HEART VALVE MONITORING AND REPLACEMENT" filed December 17, 2021, and U.S. Provisional Patent Application No. 63/291,270 entitled "SYSTEMS AND METHODS FOR PUMONARY CONGESTION MONITORING AND TREATMENT" filed December 17, 2021.

FIELD OF THE INVENTION

This invention generally relates to using cardiac signals to monitor patient health, and more specifically to using left atrial pressure signals to anticipate and treat heart conditions.

BACKGROUND

The human heart has four chambers. A left and right atrium, and a left and right ventricle. The atria receive blood from veins, and the ventricles discharge blood to arteries. The right-side structures receive deoxygenated blood from the body and send it to the lungs to be oxygenated. The left-side structures receive the newly oxygenated blood from the lungs and send it to the rest of the body. The heart similarly contains four major valves: the mitral (bicuspid) valve, the tricuspid valve, the aortic valve, and the pulmonary valve. Artificial heart valves are life-saving medical devices which can be implanted into a heart in order to replicate the functionality of natural heart valves when they fail.

Ross, J., et al: "Clinical and hemodynamic observations in pure mitral insufficiency", AMERICAL JOURNAL OF CARDIOLOGY, CARNERS PUBLISHING CO., NEWTON, MA, US, vol.2, no.1, 1 July 1958, pages 11-23, describes clinical and hemodynamic findings in patients with pure mitral insufficiency. Three methods of left atrial pressure pulse contour analysis were found useful in differentiating mitral stenosis from pure mitral insufficiency: (1) the ratio of the rate of the Y descent (i.e. the fall of the V wave) to mean left atrial pressure (Ry/MAP); (2) the ratio of the Y descent in the first 0.1 sec to the mean left atrial pressure; and (3) in patients with sinus rhythm, the ratio of the pressure at the peak of the V wave to that at the peak of the A wave (V/A).

Tice, F., et al: "Transesophageal echocardiographic assessment of reversal of systolic pulmonary venous flow in mitral stenosis", AMERICAL JOURNAL OF CARDIOLOGY, CAHNERS PUBLISHING CO., NEWTON, MA, US, vol. 75, no.1, 1 January 1995, pages 58-60, describes the use of transesophageal echocardiography and diagnostic cardiac catheterization in patients with symptomatic mitral stenosis to assess the incidence and significance of systolic flow reversal in the pulmonary veins.

SUMMARY OF THE INVENTION

A method and a system in accordance with the invention are set out in the independent claims. Particular embodiments of these aspects are set out in the dependent claims. Any subject matter contained herein that does not fall within the scope of the appended claims is considered as being useful for understanding the invention.

Systems and methods for LAP signal processing in accordance with aspects of the invention are illustrated. One aspect of the disclosure which does not form part of the presently claimed invention includes a method of treating a heart condition of a patient, including: obtaining a left atrial pressure (LAP) signal using an atrial cardiac sensor implanted into a left atrium of a patient, identifying a set of A-waves and a set of V-waves in the LAP signal, calculating an A/V ratio based on a mean pressure amplitude of the set of A-waves and a mean pressure amplitude of the set of V-waves, determining an appropriate treatment for the patient based on the A/V ratio, and providing the appropriate treatment to the patient.

BRIEF DESCRIPTION OF THE DRAWINGS

FIG. 1 is an atrial implanted cardiac monitoring system in accordance with an aspect of the disclosure.
FIG. 2 is a block diagram for a cardiac signal processor in accordance with an aspect of the invention.
FIG. 3A is a chart illustrating an example A-Wave and V-wave in accordance with an aspect of the invention.
FIG. 3B is a chart illustrating key points on the example A-Wave and V-wave in accordance with an aspect of the invention.
FIG. 4 is a flow chart illustrating a process for calculating an A/V in accordance with an aspect of the invention.
FIG. 5 is an example left atrial pressure (LAP) signal in accordance with an aspect of the invention.
FIG. 6 is an example spectral analysis of a typical LAP signal in accordance with an aspect of the invention.

FIG. 7A and 7B illustrate a LAP signal reflective of large slow respiration and shallow fast respiration, respectively, in accordance with an aspect of the invention.

FIG. 8 is a de-noised LAP signal in accordance with an aspect of the invention.

FIG. 9 is a parsed LAP signal in accordance with an aspect of the invention.

FIG. 10 illustrates the relationship between an LAP signal and an electrocardiogram (EKG) signal in accordance with an aspect of the invention.

FIG. 11 illustrates a labeled, parsed LAP signal in accordance with an aspect of the invention.

FIG. 12A and FIG. 12B illustrate a baseline V-Wave and an elevated V-wave, respectively, in accordance with an aspect of the invention.

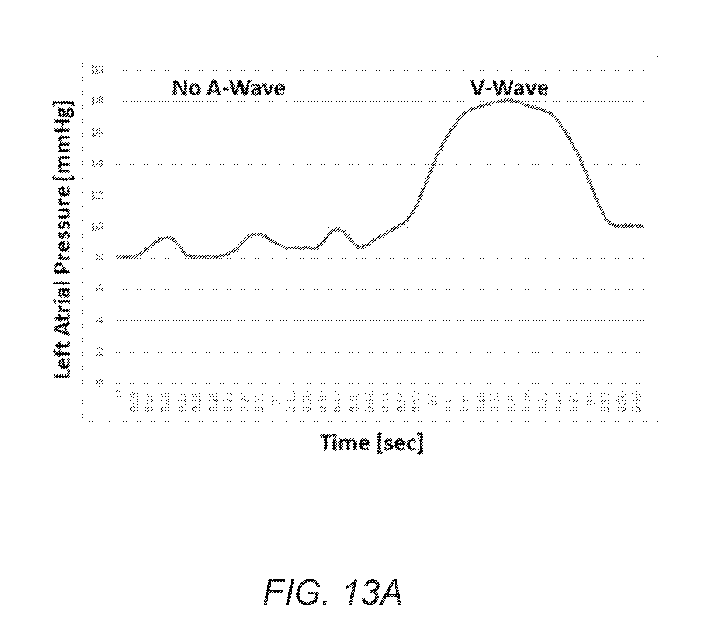

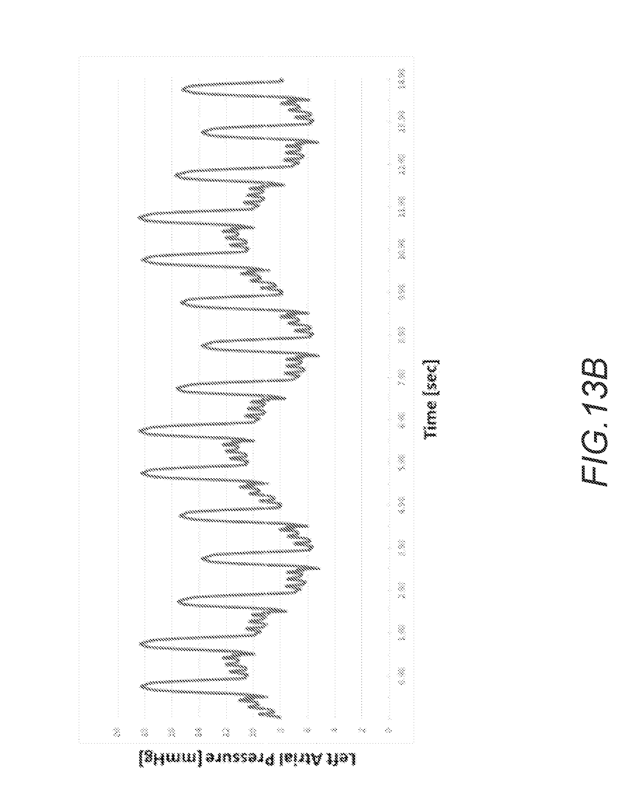

FIG. 13A and 13B illustrates a single A-V cycle, and a longer time-scale LAP signal of a patient in atrial fibrillation, respectively, in accordance with an aspect of the invention.

FIG. 14 is a flow chart illustrating a process for determining cardiac changes over different exertion states in accordance with an aspect of the disclosure.

FIG. 15 is a chart illustrating change in LAP as a function of change in heart rate in accordance with an aspect of the disclosure.

FIG. 16 is a chart illustrating change in respiration rate as a function of change in heart rate in accordance with an aspect of the disclosure.

FIG. 17 is a chart illustrating change in A/V ratio as a function of change in heart rate in accordance with an aspect of the disclosure.

FIG. 18 is a flow chart illustrating a process for dynamically presenting clinical data based on relevancy to a particular patient in accordance with an aspect of the invention.

FIG. 19 illustrates two main type of structural change during heart failure in accordance with an aspect of the disclosure.

FIG. 20 illustrates a LAP signal showing an enlarged V-wave pressure resulting from mitral regurgitation in accordance with an aspect of the disclosure.

FIG. 21 illustrates mitral valve stenosis in accordance with an aspect of the disclosure.

FIG. 22 illustrates a LAP signal showing an enlarged A-wave pressure resulting from mitral stenosis in accordance with an aspect of the disclosure.

FIG. 23 is a flow chart illustrating a process for calculating Mitral Valve Index in accordance with an aspect of the disclosure.

FIG. 24 is a chart illustrating key points on a LAP signal for calculating Mitral Valve Index in accordance with an aspect of the disclosure.

FIG. 25 is a chart illustrating a minima delta in a LAP signal in accordance with an aspect of the disclosure.

FIG. 26 is a chart illustrating a large minima delta in a LAP signal in accordance with an aspect of the disclosure.

FIG. 27 is a chart illustrating a negative minima delta in a LAP signal in accordance with an aspect of the disclosure.

FIG. 28 is a first process for calculating pulmonary congestion risk index in accordance with an aspect of the disclosure.

FIGs. 29A and 29B are charts graphically illustrating the first process on an arbitrary sample data set in accordance with an aspect of the disclosure.

FIG. 30 is a second process for calculating pulmonary congestion risk index in accordance with an aspect of the disclosure.

FIGs. 31A and 31B are charts graphically illustrating the second process on an arbitrary sample data set in accordance with an aspect of the disclosure.

FIG. 32 is a third process for calculating pulmonary congestion risk index in accordance with an aspect of the disclosure.

FIGs. 33A and 33B are charts graphically illustrating the third process on an arbitrary sample data set in accordance with an aspect of the disclosure.

DETAILED DESCRIPTION

Despite significant advancements in medical care, cardiovascular disease is a leading cause of death worldwide. Patient outcomes can be significantly more positive when underlying problems are detected earlier. Cardiac metrics such as heart rate and respiration rate are common measurements that are used in diagnostics and monitoring. In many cases, a patient's disease state can be better understood in the context of the difference in cardiac function between a resting state and an exertion state. However, cardiac function is most often measured when the patient is at rest, e.g. sitting in a doctor's office, laying on an examination table, etc. Measurement of a patient during an exertion state (e.g. moving about, walking quickly, climbing stairs, etc.) can be difficult and/or cumbersome as the monitoring equipment either needs to be able to move with the patient, or a static exertion environment with measurement capabilities must be provided.

Monitoring these metrics continuously (or even at will) at arbitrary times during the day can be difficult without the immediate assistance of a medical professional. While some modern wearable devices (e.g. smart watches) have biometric measurement functions, they can be unreliable in situations where a high degree of accuracy is required. Further, conventional wearable devices are unable to record certain pressure measurements such as (but not limited to) left atrial pressure (LAP) which as discussed herein can be used for cardiac monitoring and treatment.

To address these issues, implantable medical devices with recording capabilities have the potential to enable consistent and frequent measurement of biological functions. With implantable atrial implanted cardiac monitoring systems in accordance with various aspects of the invention, new and more accurate measurements (including LAP measurements) are now obtainable. In order to take advantage of these new data, the systems and methods described herein can record and calculate cardiac metrics which have clinical relevance for both early detection of heart disease as well as other disease states, and treatment direction. In many aspects, the derived cardiac metrics such as (but not limited to) heart rate, respiration rate, mean blood pressure, A/V ratio, etc., are of high accuracy and can be calculated over many different exertion states and/or over a long period of time in order to get an accurate picture of the difference between exertion and rest states. The changes in the ratio between the various cardiac metrics at different states can be used for diagnostics and direction of treatment.

In many aspects, atrial implanted cardiac monitoring systems can measure LAP. LAP is a measurement that has been conventionally estimated as direct measurement (e.g. via transseptal left heart catherization) is difficult and has significant associated risks. LAP can be estimated in a number of ways including during a Swan-Ganz catheterization in which a balloon-flotation technique is used to measure pulmonary capillary wedge pressure. LAP can also be estimated by measuring left ventricular end-diastolic pressure. However, these estimates can be inaccurate and can be insufficient for precision, personalized medicine.

In several aspects, implanted cardiac monitoring systems enable at-will direct recording of LAP at a high sampling rate as a continuous signal. However, merely measuring LAP is insufficient for high quality cardiac monitoring. As discussed herein, LAP signals can be processed to extract clinically significant information regarding a patient on a moment-to-moment basis and be used to determine treatment protocols for medical staff. Of particular note is the A/V ratio described herein has particular clinical relevance. For example, LAP signals on their own may not significantly change during atrial fibrillation (AF), but the derived A/V ratio can indicate the change in disease state. In particular, A/V ratio can be a useful metric when dynamics are informative, as opposed to (or as well as) absolute heart pressure values, such as in the case of measuring change in heart function between a resting state and exertion stateFor example, in many aspects, changes in A/V ratio can be used to identify and subsequently treat any number of conditions including (but not limited to) heart failure (HF), AF, hypertension, pulmonary hypertension, mitral regurgitation, mitral stenosis, aortic stenosis, and cardiac rhythm disorders (e.g. super-ventricular tachycardia, ventricular tachycardia, etc.). As can be readily appreciated, this list of conditions is not exhaustive, and any number of different conditions for which A/V ratio is clinically relevant can be analyzed as appropriate to the requirements of specific applications of aspects of the invention.

Not only are there many different types of cardiovascular disease, there are many different types of patients. As each person is at last somewhat idiosyncratic, patient outcomes can be significantly improved by personalized treatment plans. In order to rapidly generate personalized treatment plans, patients can preliminarily be classified into at least one unique class that attempts to group highly similar patients, and information can be quickly presented to medical professionals based on the classification. Further, depending on the particular classification of a given patient, various cardiac metrics may be analyzed differently. For example, normal ranges may be different for different classifications. By way of further example, certain metrics may be calculated in a different way; A/V ratio may be calculated and/or presented in a different way if a particular patient class is unlikely to show a defined A and/or V wave.

LAP can also be used to calculate heart rate and respiration rate with a high degree of accuracy using systems and methods described herein. Processes for calculation of the A/V ratio is disclosed in detail below. LAP and other cardiac signals and/or metrics in conjunction with patient information can be used by systems and methods described herein to monitor patient health and to produce personalized treatment plans. Cardiac monitoring systems are described below.

Atrial Cardiac Monitoring Systems

At a high level, cardiac monitoring systems include implanted sensors capable of generating LAP signals and processing devices capable of extracting clinically relevant information from the LAP signals. While systems that include implanted sensors are predominantly discussed below, as can be readily appreciated, processes described herein can be performed using LAP signals or estimates thereof recorded using external or temporarily implanted measurement devices.

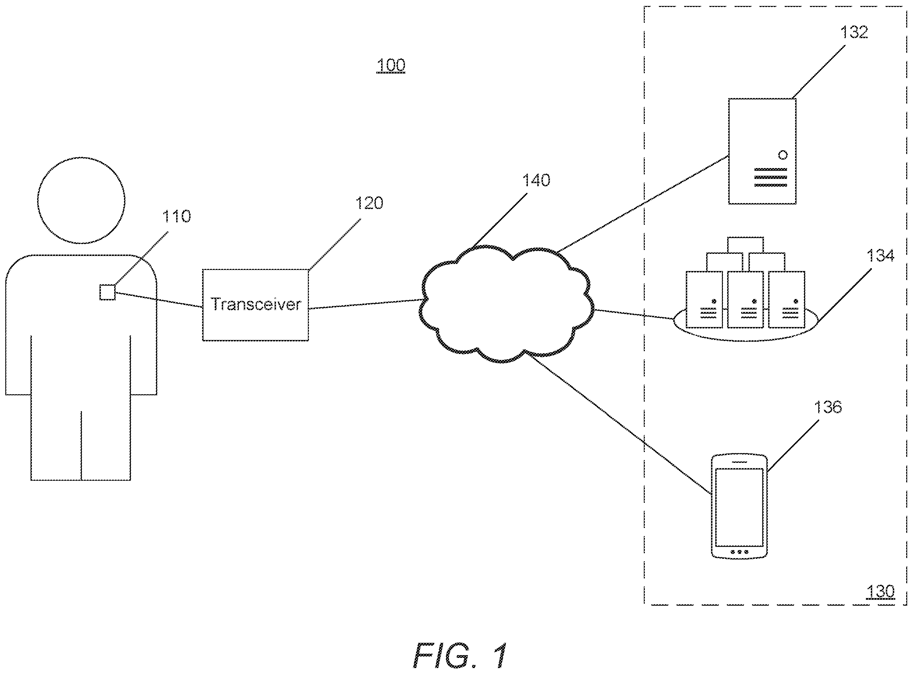

Turning now to FIG. 1, an atrial cardiac monitoring system in accordance with an aspect of the disclosure, and useful for understanding the presently claimed invention, is illustrated. System 100 includes an atrial cardiac sensor 110 implanted into the left atrium of a patient. In numerous aspects, the atrial cardiac sensor is a sensor capable of directly measuring heart function and transmitting the recorded data outside of the body. In various aspects, the atrial cardiac sensor is capable of recording a LAP signal inside the left atrium. In numerous aspects, the atrial cardiac sensor can also measure other different heart functions including (but not limited to) blood pressure, blood flow rate, heart rate, flow direction, turbulence, and/or any other cardiac metric as appropriate to the requirements of specific applications of aspects of the invention. Records of these metrics over time are referred to herein as "cardiac signals."

In many aspects, atrial cardiac sensors are capable of at least recording multiple cardiac cycles (typically at least 10-20 seconds) with a sampling rate high enough to capture the pressure waveform (typically approximately 100 Hz). In many aspects, the atrial cardiac sensor does not continuously record, and only does so when powered via a wireless power source. As such, data recorded in a single recording session may typically only record 3-25 cardiac cycles. In numerous aspects, the recording session lasts for a predetermined amount of time, e.g. between 10-60 seconds. While more cardiac cycles may be recorded via continuous powering, clinically relevant data can be extracted from data describing only this small time window.

The atrial cardiac sensor 110 can transmit recorded cardiac signals to a transceiver 120. In numerous aspects, the transceiver obtains cardiac signals from the atrial cardiac sensor using any of a number of different wireless communication methodologies. In various aspects, the transceiver can control and/or power the operation of the atrial cardiac sensor. For example, the transceiver can use wireless power transfer (e.g. radio-frequency induction) to remotely power the atrial cardiac sensor. In many aspects, powering the atrial cardiac sensor triggers the recording and transmission process. In a number of aspects, the atrial cardiac sensor has an on-board memory which stores recorded data which can later be transmitted to the transceiver. In numerous aspects, the transceiver can direct the atrial cardiac sensor to take a measurement for a predetermined period. In various aspects, the transceiver is capable of directing the atrial cardiac sensor to automatically record a predetermined number of times per day at a predetermined interval. In a variety of aspects, the transceiver further is capable of obtaining an external heart rate measurement via any of the many different types of wearable heart rate monitoring devices, and triggering the atrial cardiac sensor to take a recording in response to an elevated heart rate. Transceivers can include memory used for storing cardiac signal data received from the atrial cardiac sensor for later transmission.

The transceiver can further transmit the data obtained from the atrial cardiac sensor to one or more cardiac signal processors 130. In numerous aspects the data is transmitted via a network such as (but not limited to) the Internet. However, any number of different networks, wireless connections, wired connections, or any combination thereof can be used to transmit the data. In various aspects, the transceiver is implemented as a smart phone. In numerous aspects, the transceiver is a wearable device, such as (but not limited to), a smart watch, a chest-worn device, and/or any other wearable as appropriate to the requirements of specific applications of aspects of the invention. As can be readily appreciated, many different devices can be used as transceivers so long as they have appropriate communications and processing capabilities.

Cardiac signal processors are computing devices capable of processing measurements taken by the atrial cardiac sensor and extracting clinically relevant metrics. In numerous aspects, cardiac signal processors are capable of providing personalized treatment plans based on the clinically relevant metrics and patient information. Cardiac signal processors can be implemented any number of different computing platforms such as (but not limited to) personal computers (132), server systems (134), smart phones (136), and/or any other computing platform with sufficient computational power as appropriate to the requirements of specific applications of aspects of the invention. Further, in numerous aspects, the transceiver and cardiac signal processor can be implemented as part of the same hardware platform.

In many aspects, transceivers and/or cardiac signal processors are further capable of identifying when the atrial cardiac sensor needs replacement. In numerous aspects, unexpected changes in received signals can be used to determine the need for replacement. For example, degradation in signal quality, shifts in pressure readings over time that are not substantiated by other measurements, and/or any other change in the signal can be used as appropriate to the requirements of specific applications of aspects of the invention. In a variety of aspects, the expected lifetime of the atrial cardiac sensor can be used as part of the determination. In various aspects, the atrial cardiac sensor can transmit a signal indicating that it needs replacement. In many aspects, atrial cardiac sensors can be incorporated into artificial heart valves, and in turn can indicate when the artificial heart valve needs replacement.

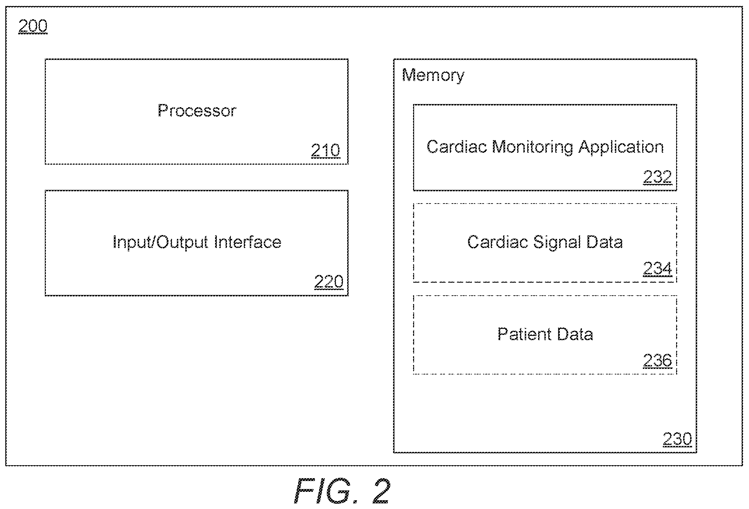

As can be readily appreciated, while a specific architecture for an atrial cardiac monitoring system is illustrated in FIG. 1, any number of different architectures (e.g. those that utilize transceivers and/or cardiac signal processors) can be utilized. Cardiac signal processors are discussed in more detail with respect to FIG. 2 below.

Turning now to FIG. 2, a cardiac signal processor in accordance with an aspect of the invention is illustrated. Cardiac signal processor 200 includes a processor 210. In many aspects, the processor is any logic processing circuit capable of carrying out cardiac monitoring processes described herein. The processor can be implemented using any (or a combination and/or multiple of) central processing units, graphics processing units, field-programmable gate arrays, application-specific integrated circuits, and/or any other logic processing circuit as appropriate to the requirements of specific applications of aspects of the invention. The cardiac signal processor 200 further includes an input/output (I/O) interface 220. The I/O interface can be any circuitry or group of circuits capable of enabling communication with transceivers and/or other computing devices. In various aspects, multiple different communication methods can be implemented using the I/O interface as appropriate to the requirements of specific applications of aspects of the invention.

The cardiac signal processor 200 further includes a memory 230. The memory 230 can be implemented using volatile memory, non-volatile memory, and/or any combination thereof The memory 230 contains a cardiac monitoring application 232 which is capable of directing the processor to carry out various cardiac monitoring processes such as those described herein. In many aspects, the memory 230 may variously contain over time cardiac signal data describing cardiac signals obtained from the atrial cardiac sensor, and patient data describing the patient about which the atrial cardiac sensor data was obtained. As can be readily appreciated, while a specific architecture for a cardiac signal processor is illustrated in FIG. 2, any number of different architectures can be utilized without departing from the scope of the invention. Cardiac monitoring processes are described in further detail below.

Atrial Cardiac Monitoring and Treatment Processes

Atrial cardiac monitoring and treatment processes in accordance with many aspects of the invention involve the utilization of cardiac signals to produce clinically actionable data. A cardiac signal of particular importance for many applications is the LAP signal, which in turn can be used to produce an A/V ratio with significant clinical relevance. The A/V ratio can be used alone or in conjunction with other cardiac signals and/or cardiac metrics to determine personalized treatment plans for a given patient. For example, in numerous aspects, if A/V ratio drops below 0.6, it can indicate that diuretics should be increased by 50%. By way of further example, if mean pressure increases above 40mmHg without a change in A/V ratio, diuretics should not be increased, and hypertension medication should be increased instead. However, any number of different treatment protocols can be used depending on the condition of the patient as appropriate to the requirements of specific applications of aspects of the invention. Further, the A/V ratio can be used as part of an early warning system for patient health, or for closed-loop/open-loop control of drug delivery. For example, changes in cardiac metrics as discussed herein can be used to automatically trigger appropriate drug delivery, e.g. via an infusion pump, or other automated delivery method. While LAP and the derived A/V ratio are not the only cardiac signals of import, processes for calculating the A/V ratio are discussed first below as a foundation.

A. Calculating A/V Ratio

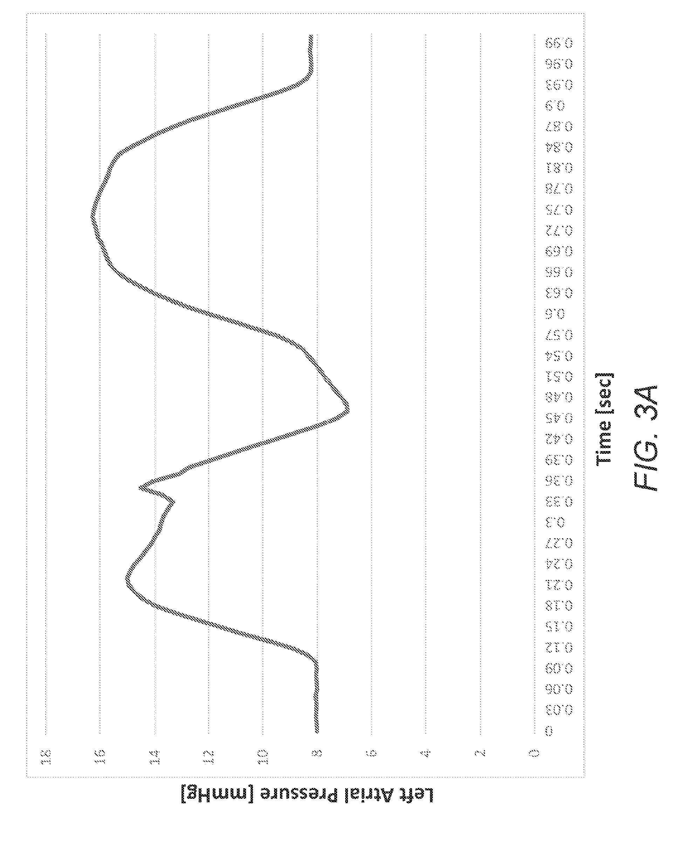

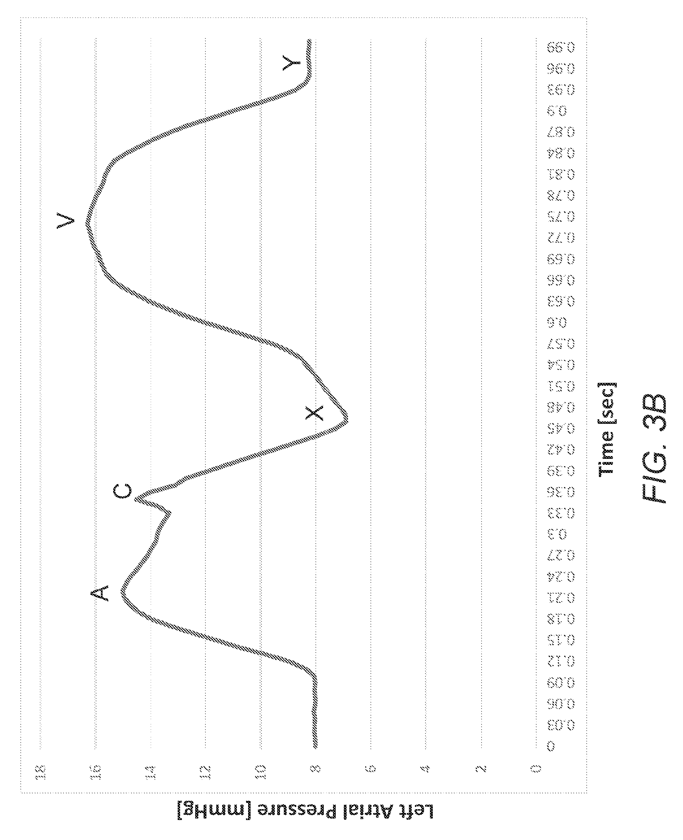

Left atrial pressure over the period of one cardiac cycle can be broken into two major features: the A-wave and the V-wave. The A-wave reflects the pressure surge due to the contraction of the atrium (sometimes referred to as presystolic or atrial systole). The V-Wave reflects the pressure surge due to blood rushing into the atrium after being pushed out of the right ventricle in systole (sometimes referred to as systolic or ventricle systole). Turning to FIG. 3A, an example LAP signal over a single cardiac cycle labeled with the A- and V-wave components in accordance with an aspect of the invention is illustrated. Additionally, five key phases can be mapped onto the LAP signal, which are indicated in accordance with an aspect of the invention in FIG. 3B. The A-wave, as noted above, indicates the right atrial contraction at the end of diastole. The C-wave indicates the mitral valve cusps bulge into the left atrium during early systole. The X descent indicates left atrial relaxation in mid systole. The V-wave again indicates the rapid filling of the right atrium in late systole. Finally, the Y descent indicates the rapid emptying of the left atrium into the left ventricle filling in early diastole.

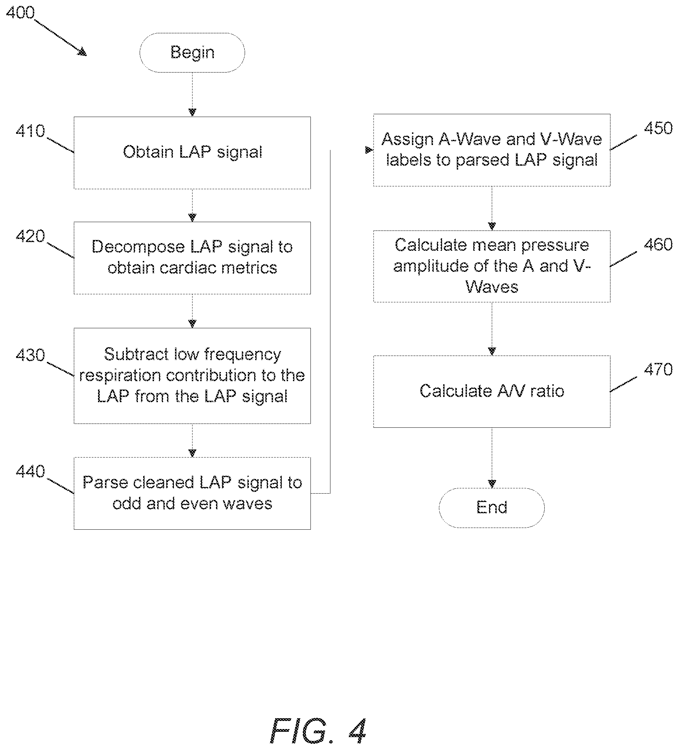

The A/V ratio is the ration between the A-wave mean amplitude and the V-wave mean amplitude. It is a very sensitive parameter because changes in the A/V ratio are earlier and more pronounced than changes in overall mean pressure in response to onset of congestion or edema. While on its face the A/V ratio might appear simple to calculate, due to constant chaos introduced by the living human body, it is in fact a non-trivial task to extract. Turning now to FIG. 4, a process for calculating A/V ratio in accordance with an aspect of the invention is illustrated

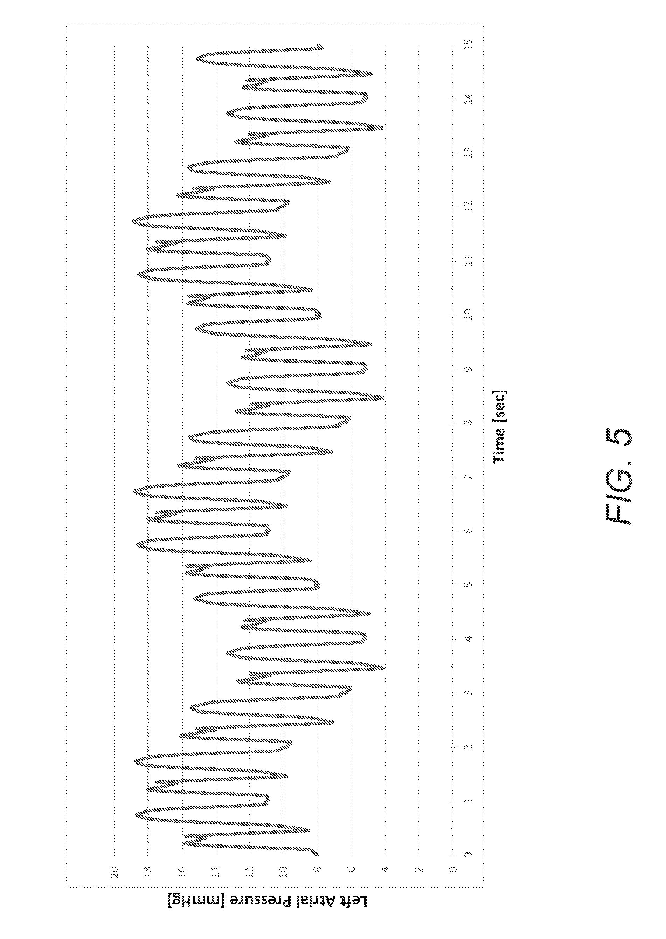

Process 400 includes obtaining (410) an LAP signal. In many aspects, the LAP signal is obtained from an atrial cardiac sensor as described above. While an LAP signal for one cardiac cycle was discussed above with respect to FIGs. 3A and B, an LAP signal over many cardiac cycles is significantly more complicated. In a real-world scenario, it is not always easy to interpret an LAP signal because there can be changes to the different LAP phases. There can also be changes due to pressure generated by respiration that contributes to LAP, typically in a lower frequency band. A real-world example LAP signal in accordance with an aspect of the invention is illustrated in FIG. 5.

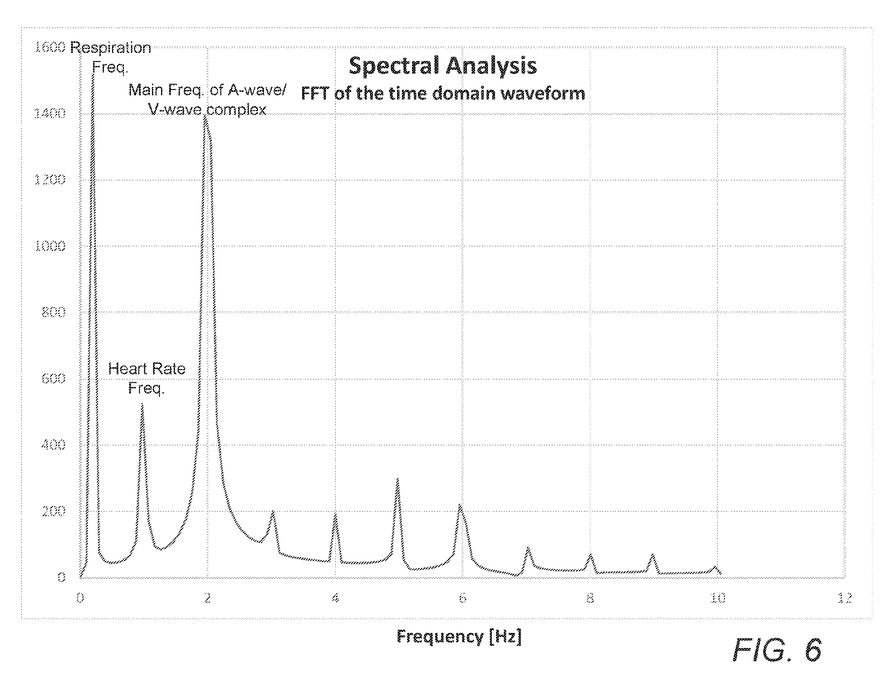

The LAP signal can be decomposed (420) as part of spectral analysis to obtain cardiac metrics. In many aspects, the LAP signal can be decomposed into frequency components using a Fast Fourier Transform (FFT). In various aspects, the LAP signal can be decomposed using wavelet analysis. The deconiposed LAP signal provides respiration frequency (typically 0.1-0.5Hz), heart rate frequency (typically 0.8-1.7Hz at rest), and the main frequency of the A-wave/V-wave complex (near 2Hz, approximately double the heart rate). Small peaks at higher frequencies typically are harmonics of the base respiration and cardiac pressure frequencies. An example spectral analysis of a LAP signal in accordance with an aspect of the invention is illustrated in FIG. 6. As can be readily appreciated, other spectral analysis methodologies that can separate respiration rate from heart rate can be used as appropriate to the requirements of specific applications of aspects of the invention. In various aspects, if respiration rate cannot be separated from heart rate, it can indicate that the patient

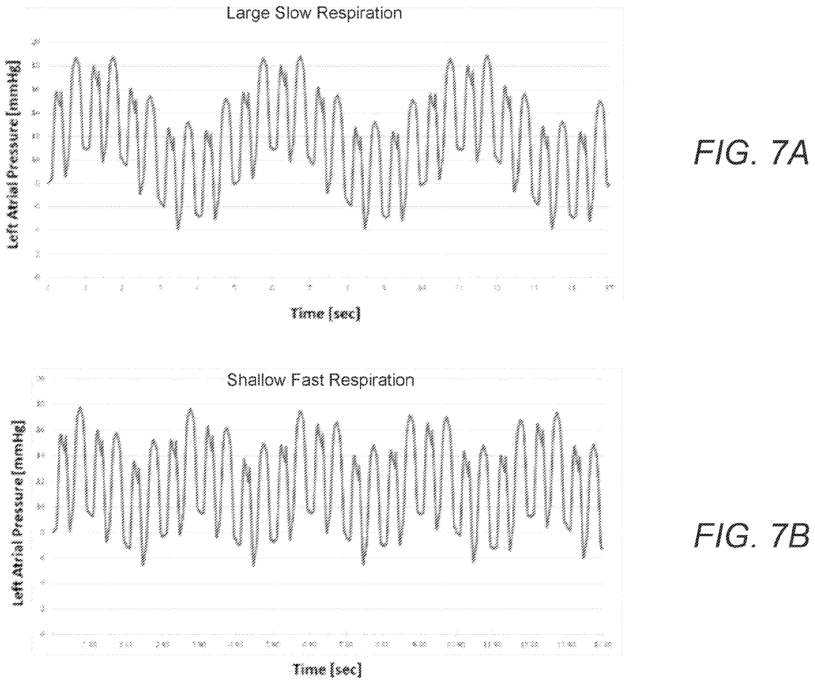

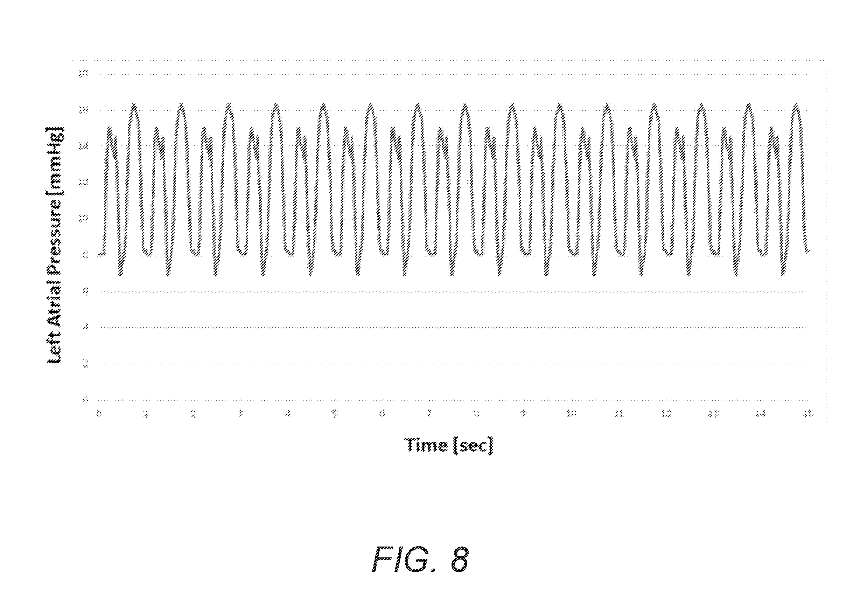

After detecting the respiration and heart rate frequencies, the respiration contribution can is subtracted (430) from the cardiac pressure component of the LAP signal by applying a lowpass filter at a frequency cutoff between the respiration frequency and the heart rate frequency. It is noted that the respiration rate and magnitude can be derived from the signal and can be important in cardiac patients. A shallower faster respiration can indicate shortness of breath which is an important symptom for a range of cardiac conditions such as pulmonary congestion and pulmonary edema. Example LAP signals with respiration components indicating large slow respiration and shallow fast respiration in accordance with an aspect of the invention are illustrated in FIGs. 7A and 7B, respectively. An example cleaned LAP signal with the respiration contribution subtracted in accordance with an aspect of the invention is illustrated in FIG. 8. Typically, heart rate starts around 40-90 beats per minute (BPM) and can go up to 220 BPM during exertion. Typical respiration rate starts at approximately 5-20 respirations per minute (RPM) and can go up to 100 RPM during exertion. However, as can readily be appreciated, these numbers are merely representative of the typical population, and may be different due to idiosyncrasies between individuals.

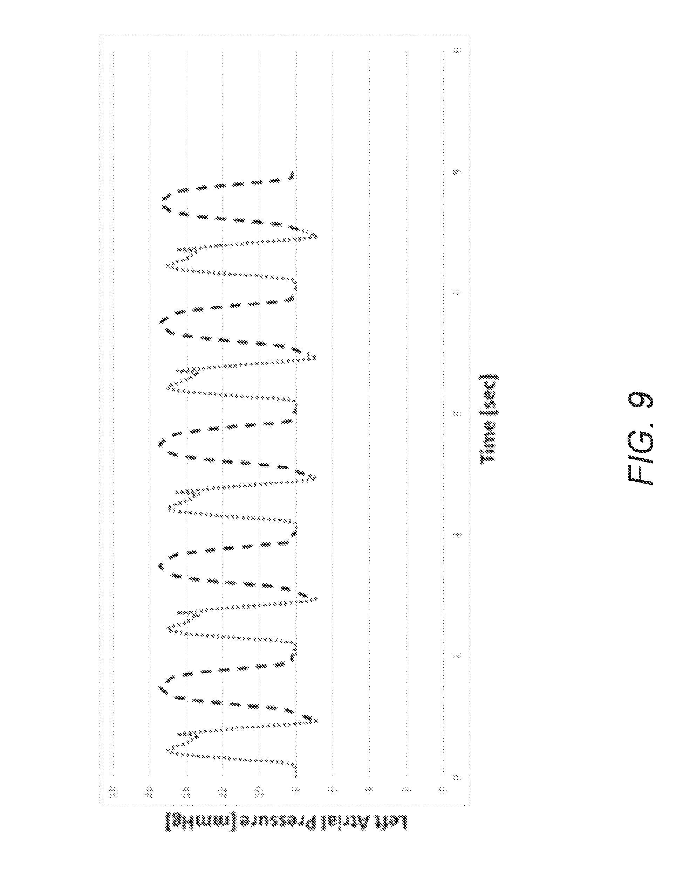

Once cleaned, the LAP signal can be parsed (440) into even and odd waves. In numerous aspects, the waveform is parsed into discrete waves at a frequency around double the heart rate. In many aspects, the local minimum in a short time window centered at double the heart rate is selected as the split point. However, any number of different methodologies can be used to split the waveform. For example, machine learning models can be used as appropriate to the requirements of specific applications of aspects of the invention. A parsed LAP signal in accordance with an aspect of the invention is illustrated in FIG. 9.

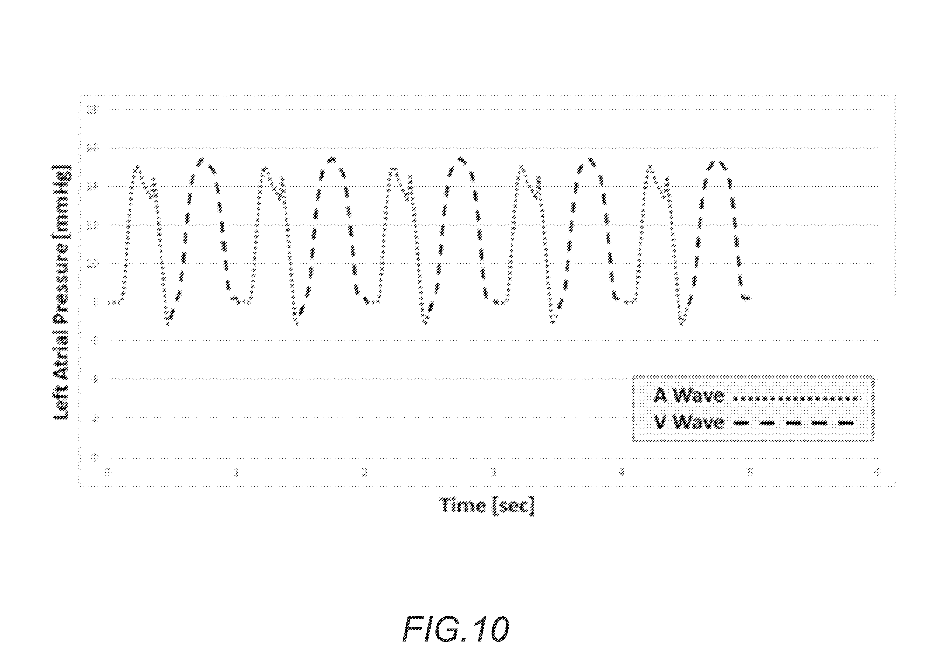

The even and odd waves can then be labeled (450) as A-waves or V-waves. A labeled version of a parsed LAP signal in accordance with an aspect of the invention is illustrated in FIG. 10. Since A and V-waves alternate, all odd waves can be assumed to belong to one group, and all even waves belong to another. A-waves can often be identified as having a double peak (A and C waves), whereas V-waves tend to have a single peak. The X descent is further typically flatter at the bottom than the Y descent, which tends to be pointier. In numerous aspects, a machine learning model can be trained and used to identify A and V-waves within a parsed LAP signal. For example, a machine learning model can be trained using a training data set made of LAP signals that have their A and V-waves annotated. However, other methodologies such as (but not limited to) searching for double peaks within a single wave can be utilized as appropriate to the requirements of specific applications of aspects of the invention. For example, in numerous aspects, for each parsed wave, if the gradient graph of the wave has two zero crossings present aside from the edges, an A wave can be assigned. If this holds true across every other wave, then the labeling can be confirmed. It is important to note that for some patients, the A-wave may not always be present. In various aspects, an inability to detect an A-wave automatically can be flagged to a medical professional for further investigation and/or treatment recommendation.

In various aspects, only a portion of the parsed signal is processed in order to increase computational efficiency. In numerous aspects, the size of the portion can be increased until a confidence threshold is reached. The wave assignments can then be extrapolated across even and odd waves in the entire parsed LAP signal. In a variety of aspects, different portions across the parsed signal can be separately processed to increase trust in the extrapolation.

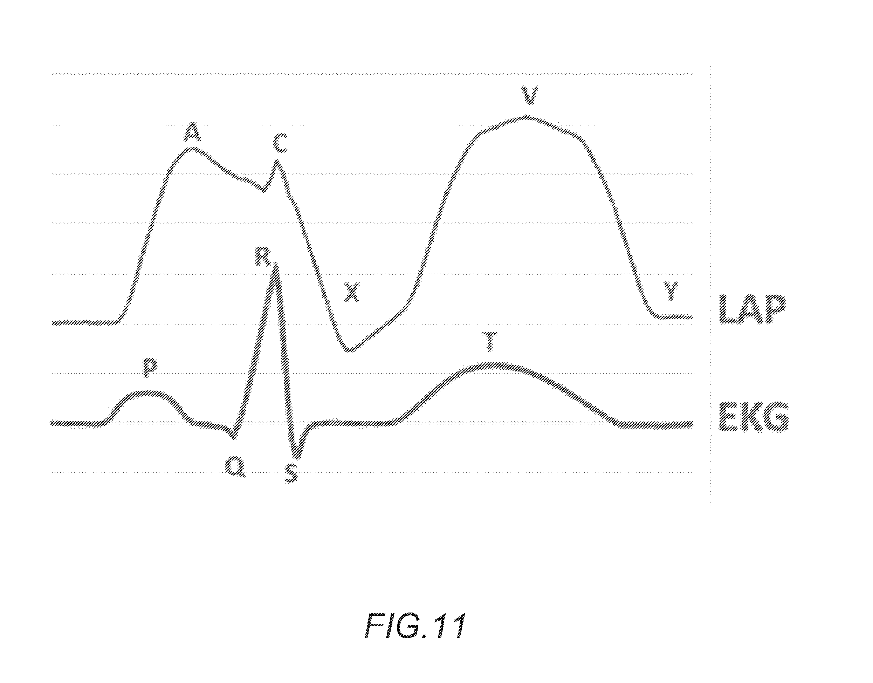

In a variety of aspects, a machine learning model that identifies A and V-waves within the parsed LAP signal can be trained using a training data set containing data collected from real cardiac condition patients together with EKG data. EKG data can be used to determine which wave is which since the A-wave typically occurs before the systole identified in the EKG as the QRS complex, and the V-wave occurs after. This relationship is illustrated in accordance with an aspect of the invention in FIG. 11. The EKG data can be used to rapidly generate sufficiently reliable labeled parsed LAP signal training data without the need for manual labeling of the training data.

Once A and V-waves have been identified, the mean pressure amplitude of the A and V-waves are calculated (460). In numerous aspects, to achieve this, the, the mean pressure and peak-to-peak pressure amplitude of all A-waves and all V-waves are calculated. The A/V ratio can then be calculated (470) as the ration between the A-wave mean amplitude and the V-wave mean amplitude.

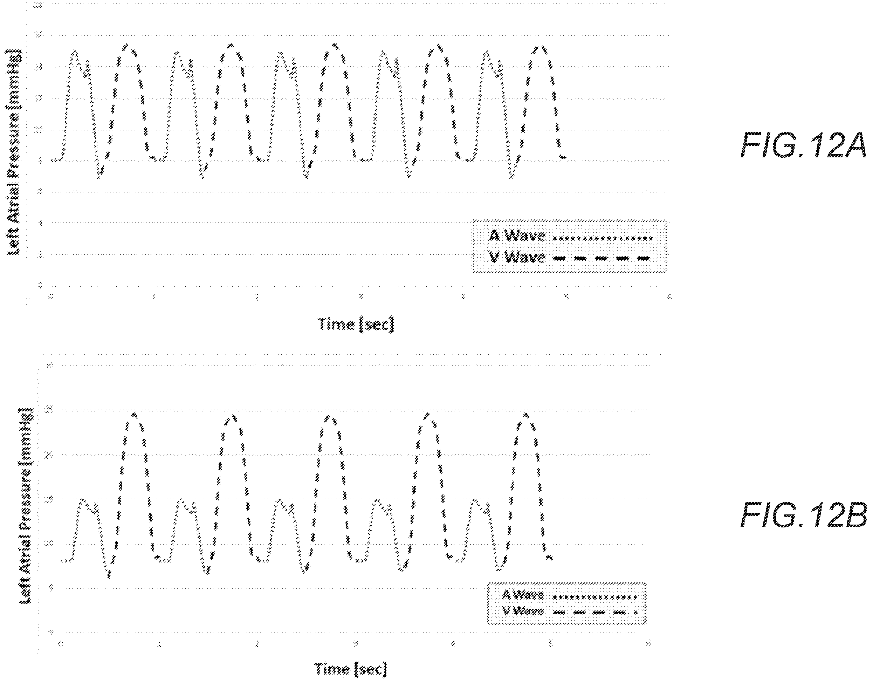

As noted above, the A/V wave is a very sensitive parameter, and therefore proper calculation is often important. To illustrate this, a parsed LAP signal with a baseline V-wave and a parsed LAP signal with an elevated V-wave in accordance with an aspect of the invention are illustrated in FIGs. 12A and B, respectively. In the illustrated example, overall mean pressure is elevated only by 20% from around 12 mmHg to around 14 mmHg, but the A/V ratio drops from around 1 to 0.4, which is a 60% change. A 2 mmHg change in LAP is generally considered to be clinically insignificant, but in this case, it can be identified as a pathological change in the V-wave uncovered by the A/V ratio. Although specific processes are discussed above with respect to the discussion of FIGs. 3-12B, any of a variety of other processes that calculate A/V ratio can be utilized as appropriate to the requirements of specific applications in accordance with various aspects of the invention.

By way of further example, elevated V-wave can be an indication of mitral valve regurgitation (MR). If the mitral valve leaks during systole, blood from the left ventricle flows back into the left atrium at high pressure. The left ventricle can then develop systolic pressures of 100-200 mmHg, which are much greater than the 10-20 mmHg LAP. MR can then be thought of as a primary cause of elevated LAP. Changes in MR can be a critical monitoring factor for treatment of cardiac conditions. For example, worsening of MR over time is an important indicator in the treatment of heart failure. MR can be caused by a number of factors including fluid retention and increased left ventricular preload. The A/V ratio can operate as a more sensitive and more specific parameter to monitor MR than LAP mean pressure, and is therefore useful in the care, monitoring, and treatment of patients with cardiac conditions.

By yet further example, some pathological changes to the A-wave can also be detected as changes in the A/V ration. The most important changes to the A-Wave, especially in heart failure patients, are typically caused by arrhythmia common to heart failure such as atrial fibrillation or atrial flutter. In these conditions the left atrium is fibrillating or fluttering instead of contracting. As such, the left atrium generates no pressure and the A-wave collapses. A collapsed A-wave in a cardiac cycle, and a LAP signal over many cycles showing collapsed A-waves in accordance with an aspect of the invention are illustrated in FIGs. 12A and B, respectively. Again, as can be seen, the overall impact on mean LAP is minimal and often masked by the elevated V-wave, but the A/V ratio is close to zero. In particular, heart failure patients can switch from normal sinus rhythm to atrial fibrillation as they decompensate. Systems that only monitor mean LAP will miss this important clinical indicator which is uncovered via the A/V ratio.

In contrast to a collapsed A-wave, a high A-wave (both alone and in A/V ratio) can suggest low tissue compliance/tamponade, constrictive pericarditis, and/or cardiomyopathy. In many aspects, sharp increases in pressure as reflected in LAP and/or A/V ratio (sudden deflection up or down) can be a critical indicator for patients in high coagulability states, as it can be an indication of pulmonary embolism. As can be readily appreciated from the above examples, the A/V ratio is a clinically significant metric. In many aspects, the A/V ratio can be calculated over time to monitor patient health. Changes in the A/V ratio can be used to uncover specific pathologies that would conventionally go unnoticed for far longer, and direct treatment towards those specific problems. As such, A/V ratio is an important metric to calculate in many scenarios. However, it is not required to calculate A/V ratio in all aspects of the invention, and many other metrics can be calculated and used by cardiac monitoring systems described herein. For example, in numerous aspects, atrial cardiac monitoring systems can monitor patients in multiple sessions over time to create a dynamic health profile. In particular, patients can be instructed to record data at different exertion states to measure cardiovascular performance.

Exertion monitoring processes are discussed further below.

B. Exertion Monitoring

Atrial cardiac monitoring systems can record measurements while a patient is ambulatory. Typically, blood pressure monitoring via implantable sensors is performed while a patient is at rest, meaning sitting down or laying down. Similar exams from non-implantable devices such as (but not limited to) pulmonary artery catheters are also conducted with the patient laying down on an exam table. Consequently, pressure signals obtained are typically limited to resting conditions. Even if the patient was previously active, recording typically takes place during a resting state. Unfortunately, some changes to pressure only occur when the patient is exerting themselves. Measuring changes during exertion states are therefore important in cardiac condition patient care. As described above, cardiac monitoring systems can include atrial cardiac sensors capable of recording measurements during exertion states, and can process data obtained at different exertion states to extract clinically relevant information.

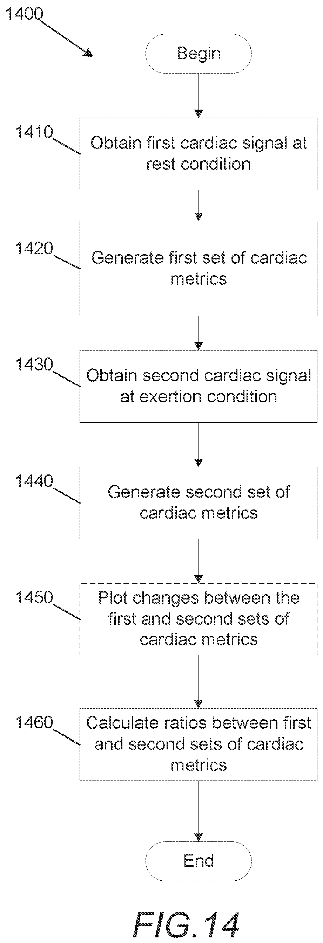

Turning now to FIG. 14, a process for exertion monitoring in accordance with an aspect of the disclosure, and useful for understanding the presently claimed invention, is illustrated. Process 1400 includes obtaining (1410) a first cardiac signal recorded while the patient is in a rest condition. In many aspects, the first cardiac signal is a blood pressure signal. In a variety of aspects, the first cardiac signal is a LAP signal. A first set of cardiac metrics are calculated (1420) from the first cardiac signal. In many aspects, the first set of cardiac metrics include heart rate, mean blood pressure, respiration rate, and A/V ratios (if the cardiac signal is a LAP signal). However, depending on the placement (and number of) atrial cardiac sensors implanted, different cardiac signals and cardiac metrics can be obtained and calculated, respectively.

Process 1400 further includes obtaining (1430) a second cardiac signal recorded while the patient is in an exertion state, and calculating (1440) a second set of the cardiac metrics. In many aspects, changes between the first set of cardiac metrics and the second set of cardiac metrics can be plotted (1450) for medical professional review. Further, ratios between the first and second sets of cardiac metrics can be calculated (1460) to derive clinically relevant information.

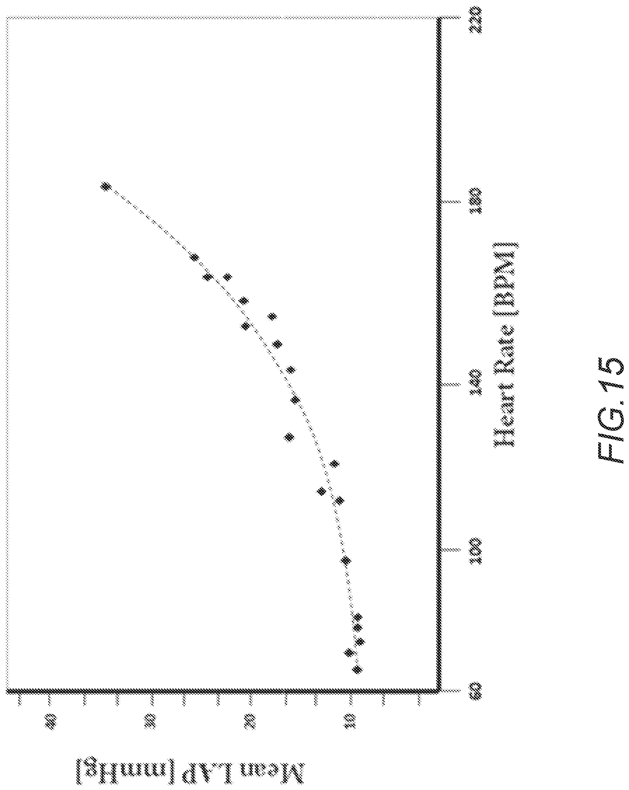

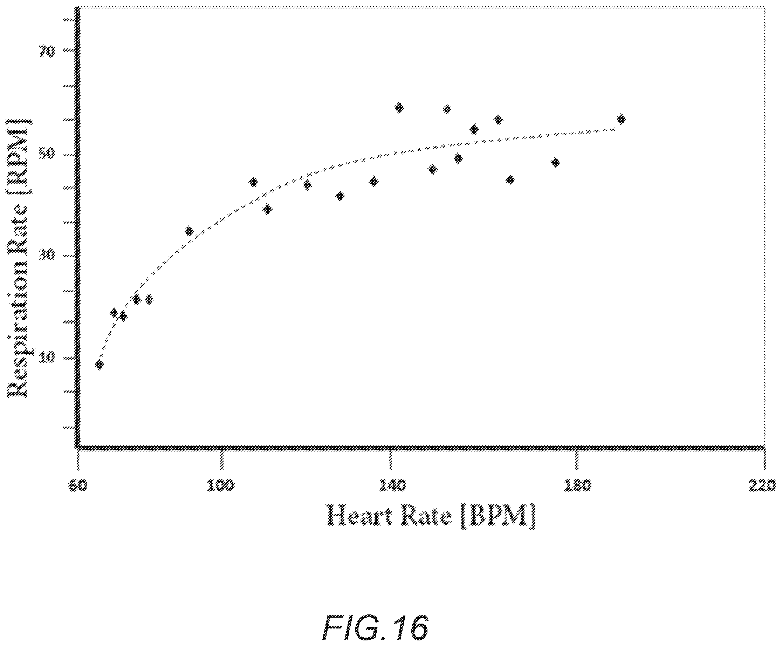

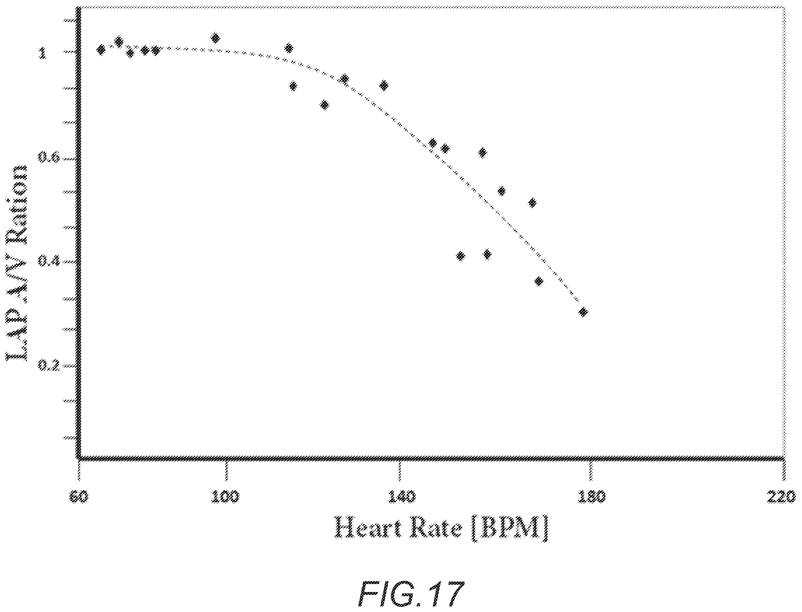

For example, in many aspects, the ratio between mean pressure change and heart rate change ($\Delta P/\Delta HR$) can be calculated. An example plot of mean LAP as a function of heart rate in accordance with an aspect of the disclosure is illustrated in FIG. 15. Further, in numerous aspects, the ratio between the respiration rate change and the heart rate change ($\Delta RR/\Delta HR$) is calculated. An example plot of respiration rate as a function of heart rate in accordance with an aspect of the disclosure is illustrated in FIG. 16. In a variety of aspects, the ratio between mean pressure change and heart rate change (A/V ratio exertion response) is calculated. An example plot of LAP A/V ration as a function of heart rate in accordance with an aspect of the disclosure is illustrated in FIG. 17. These metrics (and others) can be used to easily determine whether a patient's response to exertion is worsening, which is a clear indicator of worsening heart condition. This knowledge can be used to immediately begin treatment (or otherwise medically intervene). In numerous aspects, this process can be performed on a regular schedule (or as desired) in order to continuously check cardiac health.

LAP and/or A/V ratio can be used as (or as a component of) a heart failure index which tracks mean pressure variability over time. Tracking of heart condition can provide insight into multiple conditions including (but not limited to) heart failure, coronary artery disease, ischemia, and many others. In various aspects, exertion monitoring can supplement or even replace stress tests that are typically performed using a treadmill or pharmacological induction. As implanted cardiac sensors can collect data at any arbitrary time in a patient's day with minimal impact, tracking can happen at a more granular level than in a clinic. In clinics, typically a test is performed during a single rest state and a single exertion state which are then compared. Performance of these tests take up significant time for patients, and may not accurately reflect their normal state for any number of reasons, including (but not limited to) nervousness due to a medical setting, discrepancies between pharmaceutically induced exertion and exercise-induced exertion, and many others.

In many aspects, these data are collected and plotted on a curve. Heart rate can be used as an indicator for exertion state, and other cardiac metrics such as LAP, A/V ratio, respiration rate, etc. can be plotted over time. Portions of the graph may be colored or otherwise annotated by exertion state for easy readability. In various aspects, the curves for each metric may be labeled with warnings for metrics that are outside of healthy range, constitute unhealthy deltas between resting and exertion states, or any other annotation that maybe clinically helpful. In various aspects, the curves can be used to characterize cardiac condition and/or quantify the severity of heart disease.

While the above discussion focuses on changing heart condition across different exertion states, as can be readily appreciated, similar processes involving measurement over time irrespective of exertion condition can yield valuable information. For example, diurnal changes (night vs day) can provide clinical insight in tracking patient condition. Further, measurement of heart rate over time can enable detection of arrythmias, atrial fibrillation, supraventricular tachycardia, ventricular tachycardia, long QT syndrome, and/or any other heart-rate impacting condition as appropriate to the requirements of specific applications of aspects of the disclosure. In various aspects, heart rate patterns can be established using one or more methods, including (but not limited to) measuring A/V timing and based on the pressure morphology of the LAP signal. When other measurement tools are available, additional data can be used to confirm the veracity of the measurements. For example, a V-wave typically is expected to coincide with an echocardiogram (ECG) T-wave.

With detailed and constant access to the medical information provided by systems and methods described herein, personalized medicine becomes increasingly feasible. Indeed, systems and methods described herein can utilize data to present information differently to medical professionals based on patient profiles in order to maximize quality of care. Patient classification and personalized treatment processes are discussed further below.

C. Personalized Cardiac Healthcare

Cardiac condition patients are very heterogenous, and similar cardiac signals can mean different things in different patients. As discussed throughout, various systems and methods described herein can obtain a wide variety of cardiac signals at will and generate clinically relevant cardiac metrics that have increased sensitivity for each patient. Systems and methods described herein can be used to classify patients to any of a number of subcategories, and cardiac signals and metrics can be presented to the medical professionals in a context that best fits the patient's overall classification.

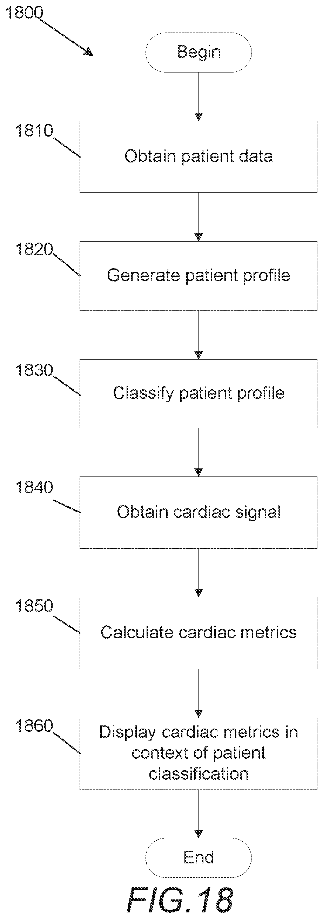

Turning now to FIG. 18, a process for personalized presentation of cardiac information in accordance with an aspect of the disclosure, and useful for understanding the presently claimed invention, is illustrated. Process 1800 includes obtaining (1810) patient data. In numerous aspects, patient data includes background patient data. Background patient data can be manually entered by a healthcare professional and/or obtained via electronic health records. The patient can then be parameterized based on their background patient data to generate (1820) a patient profile. Parameters for a patient profile can include (but are not limited to): sex; type of cardiac condition (cardiac condition with preserved ejection fraction (diastolic) - HFpEF; cardiac condition with reduced ejection fraction (systolic) -HFrEF); hypertension (none; mild; severe); pulmonary hypertension group (Group I - primary PH (idiopathic); Group II - secondary PH (driven by elevated LAP); Group III - active lung disease; Other); arrhythmia (none; paroxysmal atrial fibrillation; persistent atrial fibrillation; other); mitral regurgitation (mild; moderate; severe); other valvular pathologies (regurgitation; stenosis); ischemia and/or post-ischemia scar tissue; pacemaker (yes; no); and/or any other relevant parameters as appropriate to the requirements of specific applications of aspects of the disclosure.

Patients are classified (1830) based on the parameterized patient profile. In numerous aspects, each unique combination of parameter values can be treated as a separate class. In numerous aspects, the patient profile is provided to a machine learning model which produces a classification of the input profile. Classification using machine learning is discussed in further detail below. Patients in each class present more homogenously, and it can be assumed that the LAP waveform for each patient in that class can be interpreted in the same or a similar way. One or more cardiac signals are then obtained (1840) and cardiac metrics are calculated (1850). In numerous aspects, depending on the classification of the patient from which the cardiac signal was recorded, the way cardiac metrics are calculated can differ to be more appropriate to the type of heart disease the patient presents with. The cardiac metrics can then be displayed (1860) to medical professionals in context of the classification via a user interface. In numerous aspects, the classification is presented with the metrics. In various aspects, different ranges for normal/abnormal may be presented for one or more cardiac metrics based on the classification. In a variety of aspects, different cardiac metrics may be highlighted as more likely to be clinically relevant based on the classification. In many aspects, different treatment methods may be suggested based on the cardiac metrics and the classification. As can be readily appreciated, any number of different personalized medicine applications can be implemented. Turning now to classification using machine learning, several viable alternatives are presented. In many aspects, a training data set which includes patient profiles annotated with classifications is generated and used to train a supervised machine learning model. In various aspects, the classifications are assigned respective normal cardiac metric ranges. Provisioning of a patient profile to the trained machine learning model can produce a classification of said patient profile, which in turn can be used to interpret the cardiac metrics.

In numerous aspects, the supervised machine learning model which is trained is a random forest model, or a support vector machine. In the case of the random forest model, multiple decision trees can be generated to produce a final model prediction. In contrast, the support vector machine can maximize the boundary between different classes that maximizes the margin between them. In various aspects, more than one model is used. When more than one model is used to classify a patient profile, both classifications may be provided. In some aspects, a definitive classification is only provided if the outputs of the models agree. If there is disagreement, this can be flagged to the medical professional. Further, classification confidence values can be provided signifying the classification probability produced by the model. If the confidence value is below a predetermined threshold (e.g. between 75-99%, although this number can be tuned to the risk comfort of the user), then no positive classification may be provided.

As can readily be appreciated, other supervised machine learning models can be used to produce classifications. In various aspects, an unsupervised machine learning model is used in order to eliminate the burden of generating training data. While in some cases accuracy may be lower, unsupervised learning enables an unbiased classification process which may reveal new insights. For example, a clustering approach can be used on agglomerated patient data into which the instant patient profile is introduced.

In many aspects, when machine learning is utilized for classification, the patient's LAP signal and/or derived cardiac metrics may be incorporated into the patient profile and provided to the machine learning model in order to further assist with the classification process. In various aspects, dimensionality reduction may be applied based on waveform features prior to provisioning the machine learning model.

Utilization of machine learning for classification can be particularly useful when integrated into an electronic health record (EHR) system. The EHR may already consist of patient profiles annotated with patient outcomes and diagnoses. In this way, the EHR itself can be used as a training data set, or as the agglomerated set of patient profiles in the case of unsupervised learning. With a sufficiently well-populated EHR, classification can rise to the level of disease cluster or even individual disease level classification, over even healthy cardiac metric ranges. In some aspects, healthy cardiac metric ranges can be further derived in the context of an EHR by observing the range of cardiac metrics at discharge across the EHR population for that particular class.

D. Implant Monitoring

Overall cardiac health monitoring remains is important function. For many patients who are fitted with an atrial cardiac sensor, additional implants may be (or already be) implanted to maintain cardiac health. However, implanted cardiovascular medical devices may deteriorate over time or be damaged or otherwise impaired via accident. LAP and/or A/V ratio can be used to measure the functionality of implantable medical devices such as shunts and/or heart valves. In numerous aspects, the pressure difference between different sides of a shunt or valve can be used to build a pressure profile, which in turn is suggestive of blood flow. Measurements can be taken using a sensor implanted on either side of the shunt/valve, or via a sensor on one side of the shunt/valve and an external measurement modality. In some aspects, valves and/or shunts may include cardiac sensors capable of pressure measurements as described herein on one or both sides capable of making the requirement measurements.

For example, mitral valve diseases are common forms of cardiovascular disease. There are two main mitral valve abnormalities: mitral regurgitation and mitral stenosis. Mitral valve diseases are often contributing factors of heart failure. In many aspects, mitral valve disease state can be monitored through the use of a Mitral Valve Index (MVI) which reflects the degradation of the mitral valve. MVI can be calculated based on LAP measurements over time, and updated as new LAP measurements are taken. When the MVI exceeds a prespecified level, systems and methods described herein can be used to alert that a mitral valve intervention is needed (e.g. surgical percutaneous valve repair). In numerous aspects, artificial heart valves are monitored using MVI. If the artificial heart valve begins to degrade, MVI can similarly be affected and used to trigger an alert and subsequent repair or replacement.

As can be readily appreciated, while sensors predominantly configured to be implanted in an atrium are discussed above, similar sensors can be made which measure pressure in other regions of the circulatory system, and therefore such pressure differentials can be obtained outside of the left atrium. In many aspects, a mitral valve index is calculated in order to monitor implant status and/or overall cardiac health.

Mitral valve disease can lead to changes in pressure and flow in the left atrium. As noted above, there are two main mitral valve abnormalities: mitral regurgitation and mitral stenosis. In mitral regurgitation, the valve fails to close properly and there is a reverse leak of blood from the left atrium backwards into the left ventricle. Since the ventricle develops much higher pressure than the atrium, the regurgitation causes a pressure spike in the atrium with timing corresponding to the ventricular systole. In numerous aspects, on a LAP signal this appears as an elevated V-wave. Mitral regurgitation is common in heart failure patients.

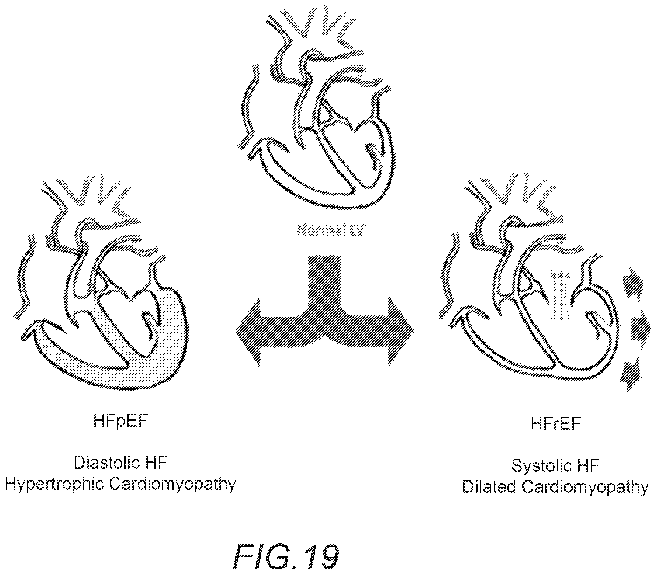

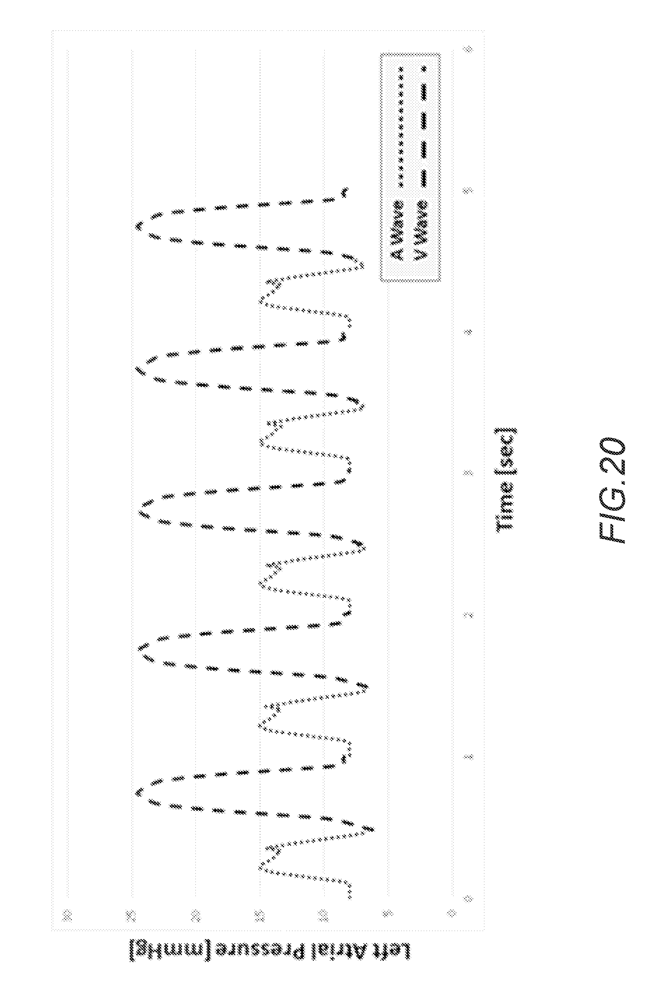

The two main types of heart failure that are associated with two different structural changes in the heart: Heart Failure with reduced Ejection Fraction (HFrEF) and Heart Failure with preserved Ejection Fraction (HFpEF). About 65% of HF patients are HFrEF patients. They have an enlarged heart and reduced Ejection Fraction. If the heart is severely enlarged the mitral valve leaflets are pulled away from each other and fail to close. The remaining 35% of HF patients are HFpEF patients. The heart ventricle muscle walls are thicker than normal, fibrotic, and stiff. The mitral valves typically do not leak in HFpEF, but the stiff ventricle creates a diastolic disfunction in which the ventricle filling by the atrium is longer and generates higher pressure in the atrium. A graphical depiction of the different structural changes in HFrEF and HFpEF hearts compared to normal is illustrated in FIG. 19. Systems and methods described herein can parse LAP signal waveforms to diagnose both HFrEF and HFpEF, and tell the difference between them. In HFrEF mitral valve regurgitation tends to be more common, and presents as a high V-wave (e.g. a peak V-wave LAP of over 25 mmHg) even in moderate mean LAP values (5 - 25 mmHg). In HFpEF, both the A-Wave and the V-Wave tend to be elevated, and therefore the mean LAP is more elevated (e.g. above 20 mmHg) while the V-Wave is also moderately elevated (e.g. also around 20 mmHg). By way of example, a LAP signal showing enlarged V-wave pressure resulting from mitral regurgitation in accordance with an aspect of the disclosure is illustrated in FIG. 20. It is important to note that aortic stenosis can also cause elevated A-wave pressure.

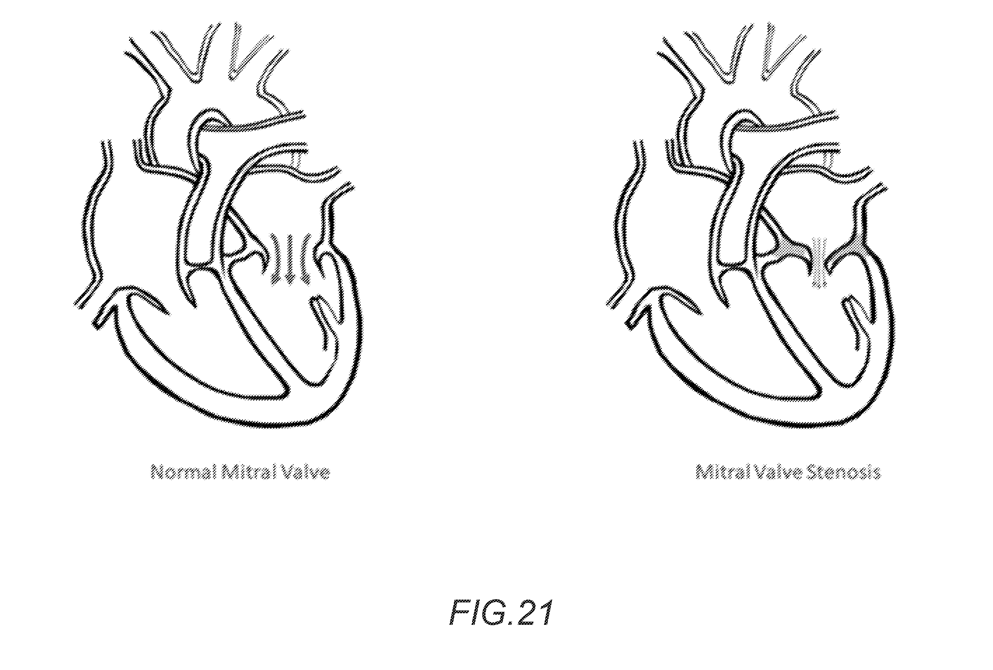

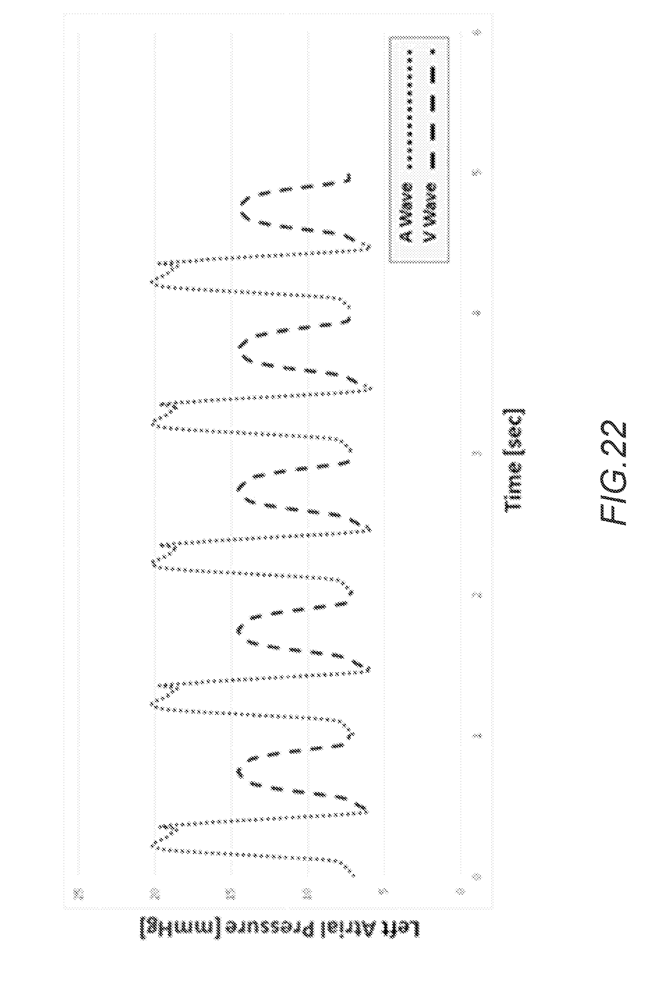

Mitral stenosis can occur when the valve is diseased, calcified, or otherwise enlarged with thicker leaflets, and fails to fully open. During atrial contraction, the mitral valve normally is open wide to allow blood to flow into the ventricle. A stenosed valve opens only slightly, and therefore the pressure in the atrium is elevated. A graphic depiction of a normal mitral valve compared to mitral valve stenosis is illustrated in FIG. 21. In a LAP signal, this is reflected by an elevated A-wave. An example LAP signal with an elevated A-wave due to mitral valve stenosis in accordance with an aspect of the disclosure is illustrated in FIG. 22.

In order to detect and monitor mitral regurgitation and/or mitral stenosis, cardiac sensors as described herein can be used to record LAP signals. Multiple LAP signals can be taken over time and be analyzed to determine the health of a patient's mitral valve. In numerous aspects, cardiac metrics can be used to quantify the severity of the mitral valve state. In many aspects, a Mitral Valve Index (MVI) is calculated using A/V ratio and the minimum pressure point between the A-wave and the V-wave. When the MVI reaches above a threshold value, an indicator can be provided that intervention is necessary. In many aspects, the intervention is a surgical percutaneous valve repair. In some aspects, the intervention is a surgical percutaneous valve replacement. In various aspects, the intervention is the replacement of an artificial heart valve.

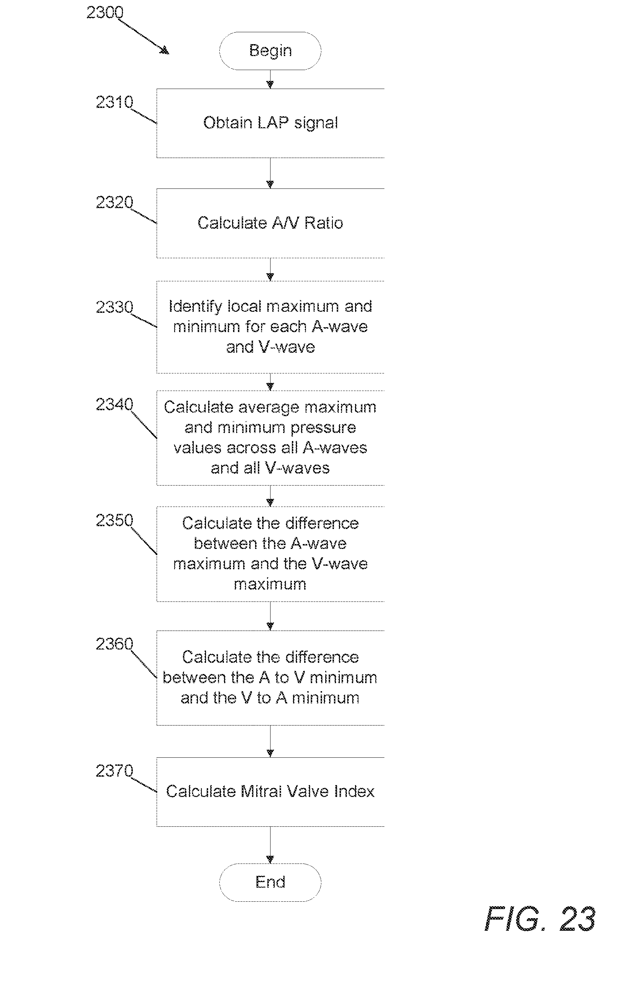

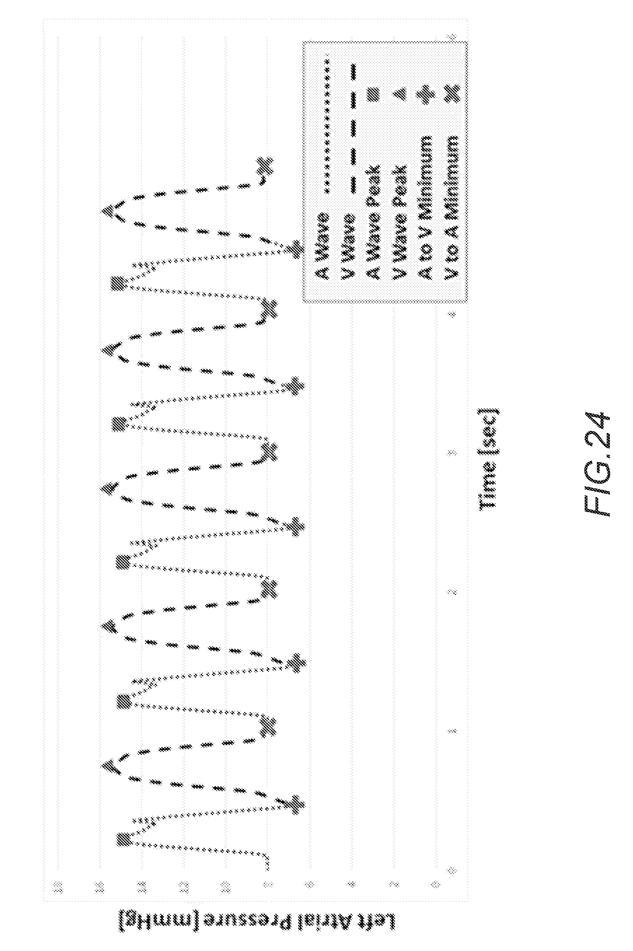

Turning now to FIG. 23, a process for calculating MVI in accordance with an aspect of the disclosure, and useful for understanding the presently claimed invention, is illustrated. Process 2300 includes obtaining (2310) a LAP signal. In numerous aspects, multiple LAP signals are obtained. From the LAP signal(s), A/V ratio is calculated (2320). During the A/V ratio calculation, A-waves and V-waves are identified. For each A-wave and V-wave, a local maximum and minimum are identified (2330). In numerous aspects, the minima occur at the A to V, and V to A transitions. The minima at the A to V wave transition is sometimes referred to as the "X depression". Similarly, the minima at the V to A wave transition is sometimes referred to as the Y depression. In various aspects, the maxima occur at the peaks of the respective wave. An example normal LAP signal marked with minima and maxima in accordance with an aspect of the disclosure is illustrated in FIG. 24. Typically, the A to V transition minimum is slightly higher than the V to A transition minimum.

The average maximum pressure and average minimum pressures are calculated (2340). The difference between the average A-wave maximum and the average V-wave maximum is calculated (2350), ΔMax.

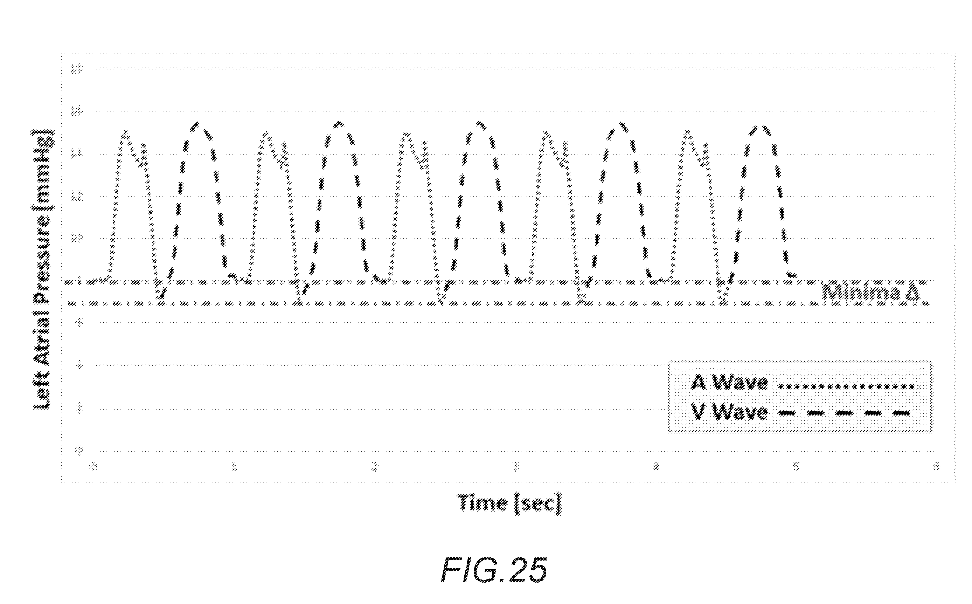

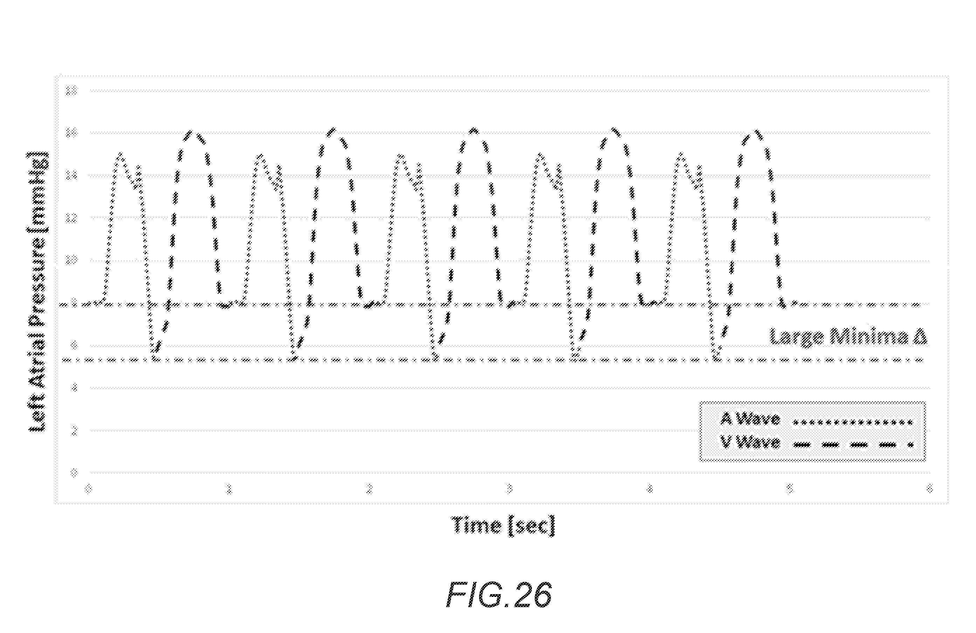

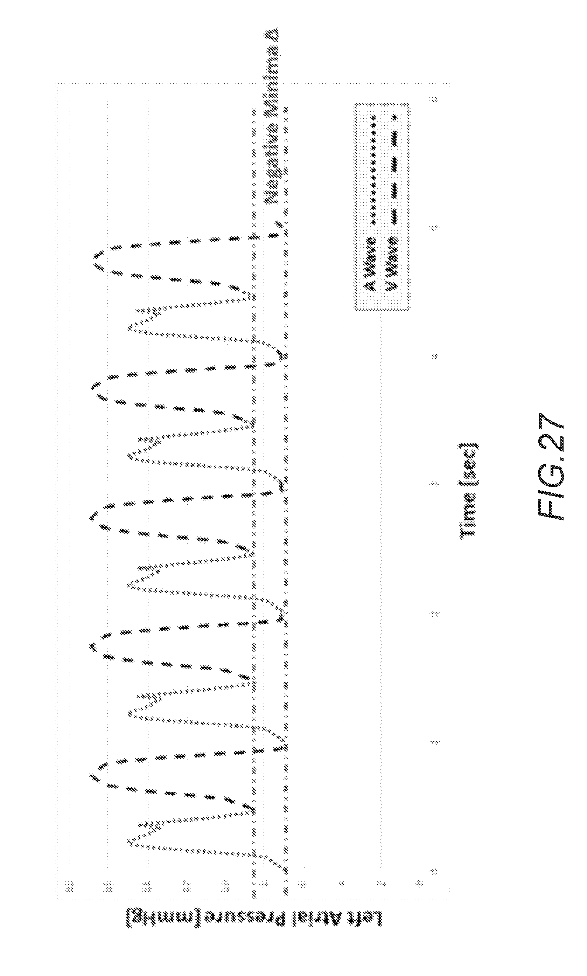

The difference between the average A to V minimum and V to A minimum is calculated (2360), ΔMin. In numerous aspects, an increased ΔMin can indicate a diastolic disfunction. An example normal ΔMin in accordance with an aspect of the disclosure is illustrated in FIG. 25. In contrast, an example increased ΔMin in accordance with an aspect of the disclosure is illustrated in FIG. 26. In various aspects, an inverted ΔMin (e.g. a negative ΔMin resulting from a greater average V to A pressure compared to average A to V pressure) can indicate a late contraction of the ventricle in conditions such as heart block or long QT syndrome. An example negative ΔMin in accordance with an aspect of the disclosure is illustrated in FIG. 27.

The A/V ratio, ΔMax, and ΔMin are then factored together to calculate (2370) the MVI. In many aspects, MVI can be calculated as:

$$MVI = (A/VRatio)(\frac{C1}{\Delta Max})(\frac{C2}{\Delta Min})$$

where C1 is the ΔMax of a healthy heart, and C2 is the ΔMin of a healthy heart. Typically, C1 and C2 are between 1 and 2, however, C1 and C2 values can often range between 1 and 5. In numerous aspects, the MVI is used to determine an appropriate time for surgical intervention. In many aspects, an MVI > 1 is considered normal, whereas an MVI < 1 can suggest a need for intervention. In various aspects, MVI is calculated using LAP signals recorded during a patient rest state, and calculated again using LAP signals recorded during a patient exertion state. In some aspects, MVI calculations involve using both A/V ratio and ΔMax/ΔMin values calculated during both exertion and rest states. In a variety of aspects, respiration rate and respiration pressure magnitudes (which can be derived from LAP as discussed above) are also used in conjunction with MVI to monitor cardiopulmonary states of a patient. Changes in lung effect pressure can be monitored and used to track and treat lung conditions. As can be readily appreciated, while the above is discussed with specific reference to LAP, in many aspects, cardiac sensors can equally be implanted in the right atrium and the right atrial pressure (RAP) can be analyzed to detect and monitor tricuspid valve abnormalities.

E. Cardiopulmonary Monitoring

heart failure (HF) is a condition where a patient's heart is impaired and cannot generate enough blood output to supply the demand of the patient's organs for oxygen. When this condition becomes chronic, permanent changes appear in the heart structure and hemodynamics which often result in chronic symptoms that impair quality of life. The most common symptom is pulmonary congestion, leading to HF often being referred to as congestive heart failure (CHF).

Pulmonary congestion is a result of pulmonary hypertension and poor blood flow through the lungs.

Heart failure elevates the blood pressure in the heart left atrium which drains blood from the lungs. When left atrial pressure (LAP) is elevated, the blood pressure in the lungs is also elevated, and cause fluid to defuse from the blood capillaries in the lungs to the air cavities (alveoli) in the lungs, causing congestion. In severe pulmonary congestion, the patient is effectively drowning and cannot get enough air into their lungs. This often requires hospitalization to treat.

Treatments include medication to try and minimize pulmonary congestion. Medications include diuretics to reduce general fluid volume of the patient and antihypertension drugs to lower blood pressure. High dosages tend to negatively impact quality of life by causing frequent urination and hypotension related fatigue. Accordingly, optimizing medical management is key to maintaining quality of life for HF patients.

Conventionally, patients monitor their heart failure by monitoring blood pressure, heart rate, pulse, weight, and how they feel (e.g., energy level, clothes feeling tight, swelling, shortness of breath, coughing, etc.). These are often subjective and can be difficult for patients to express and doctors to understand. Further, as noted above, conventional wearable devices are unable to record certain pressure measurements such as (but not limited to) left atrial pressure (LAP) which as discussed herein can be used for cardiopulmonary monitoring and treatment. Patient outcomes can be significantly more positive if worsening of pulmonary congestion could be predicted so it could be more aggressively treated, for example by increasing drug dosage before congestion gets too severe that it requires hospitalization.

In many embodiments, a pulmonary congestion risk index is calculated as the area under the mean LAP signal curve above a hypertension level (sometimes also referred to as a "LAP threshold"), in order to monitor a patient. In numerous aspects, at least one LAP signal is recorded daily. Each day, the new LAP signal is analyzed in conjunction with previously recorded daily LAP signals for the patient to trend the data and determine the risk of developing pulmonary congestion. The risk can be represented by a pulmonary congestion risk index. Further, due to noise in the LAP signals and/or the temporal choppiness of their acquisition, filtering and/or averaging techniques can be applied. When the pulmonary congestion risk index exceeds a threshold value, an alert can be provided which can trigger treatment of the patient to prevent severe congestion.

Congestion is caused by diffusion of fluid from the blood to the lung cavities. The speed of diffusion is controlled by the pressure gradietit from the blood to the air cavities. If LAP is elevated then pulmonary blood pressure is also elevated, and fluid buildup in the lungs is faster. Excess fluid is usually slowly evacuated from the lungs by reabsorption and exhalation. It the fluid buildup rate is higher than the fluid evacuation rate, fluid builds up in the lungs and can lead to severe congestion. Therefore, integrating elevated pressure over time can be a predictor of pulmonary congestion.

Pulmonary congestion risk indices are a measure of the severity and length of time for which a patient's mean LAP exceeds a hypertension level In numerous aspects, the hypertension level is 20 mmHg which can be considered as high pressure in the left atrium. But chronic HF patients who are medicated can sustain much higher pressures and their physician may choose a higher threshold. Similarly, some patients may benefit from a lower threshold which reflects high pressure for their heart depending on their particular condition. An index time window can be predetermined for the analysis, typically between three and thirty days. The pulmonary congestion risk index can be calculated by finding all instances in the time window in which the daily mean LAP is above the threshold and summing the differences between the mean LAP and the threshold for all instances in the time window.

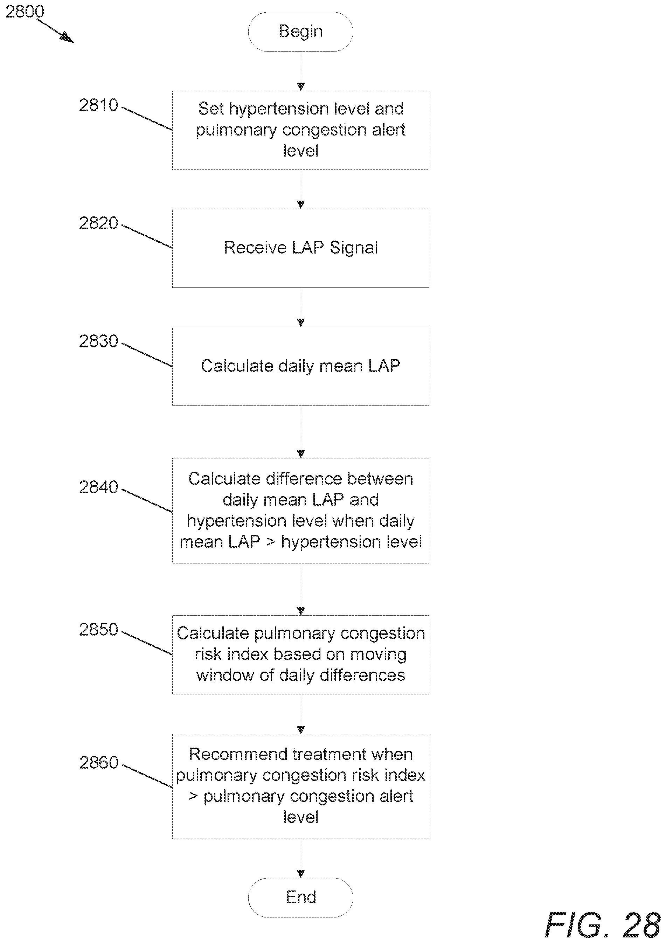

Turning now to FIG. 28, a first process for calculating pulmonary congestion risk index in accordance with an aspect of the disclosure, and useful for understanding the presently claimed invention, is illustrated. Process 2800 includes setting (2810) the hypertension level and pulmonary congestion alert level. In numerous aspects, when the hypertension level is 20 mmHg, the alert level is set at 30 mmHg. The alert level can be set to any arbitrary value depending on the specific patient. In numerous aspects, the hypertension level is 20 mmHg, although this number can be varied depending on the particular patient as discussed above. A LAP signal is received (2820) on a daily basis, and the daily mean LAP is calculated (2830) for each day. In many aspects, the LAP signal is recorded by an atrial cardiac sensor. The LAP signal can be received at a cardiac signal processor via a transceiver as discussed above. In numerous aspects, multiple LAP signals are recorded every day and are averaged together. In numerous aspects, LAP signals are recorded daily during a similar exertion state, e.g., during rest.

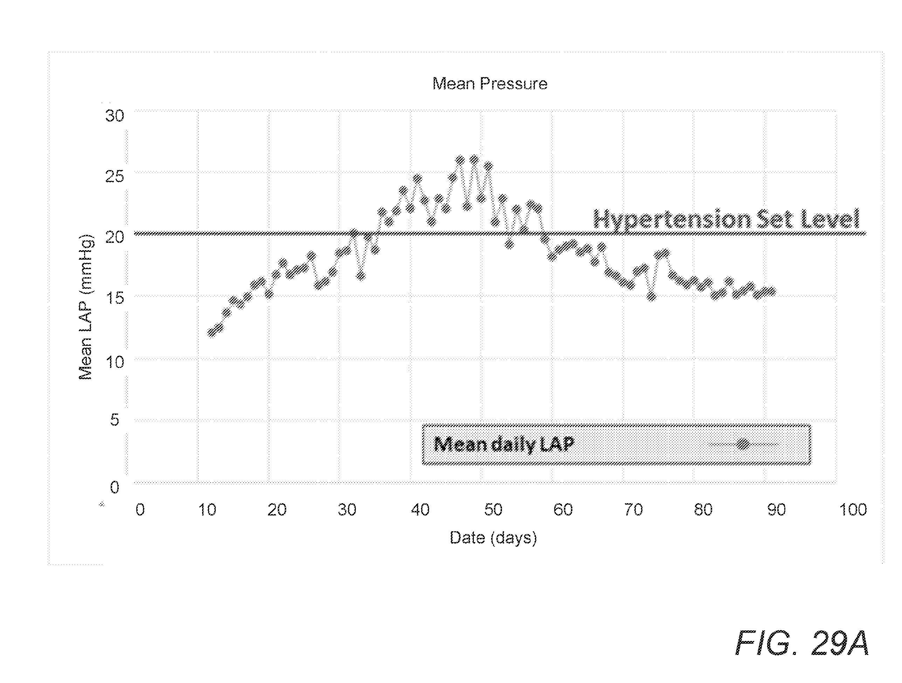

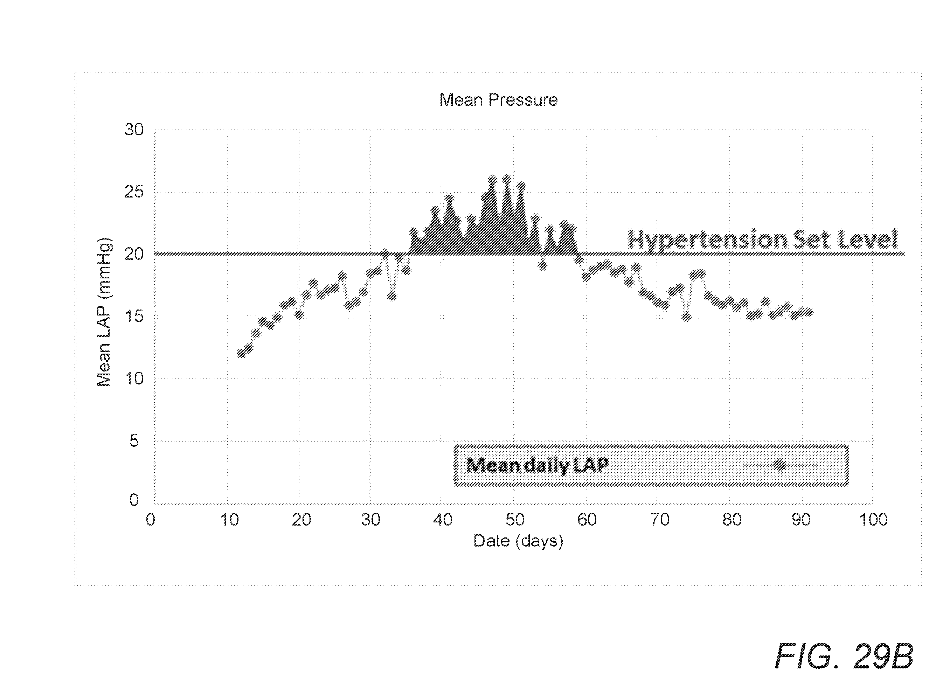

When the daily mean LAP for a particular day is greater than the hypertension level, then the difference between the daily mean LAP and the hypertension level is calculated (2840). The pulmonary congestion risk index is then calculated (2850) by summing the daily differences over the moving index time window. If the pulmonary congestion risk index exceeds the pulmonary congestion alert level, then an alert is triggered, and a treatment can be recommended (2860), and in many aspects, performed. Turning now to FIG. 29A and 29B, process 2900 is graphically illustrated on an arbitrary set of sample data in accordance with an aspect of the disclosure. FIG. 29A illustrates the mean LAP over time, and the newly shaded region of FIG. 29B shows the value of the pulmonary congestion risk index.

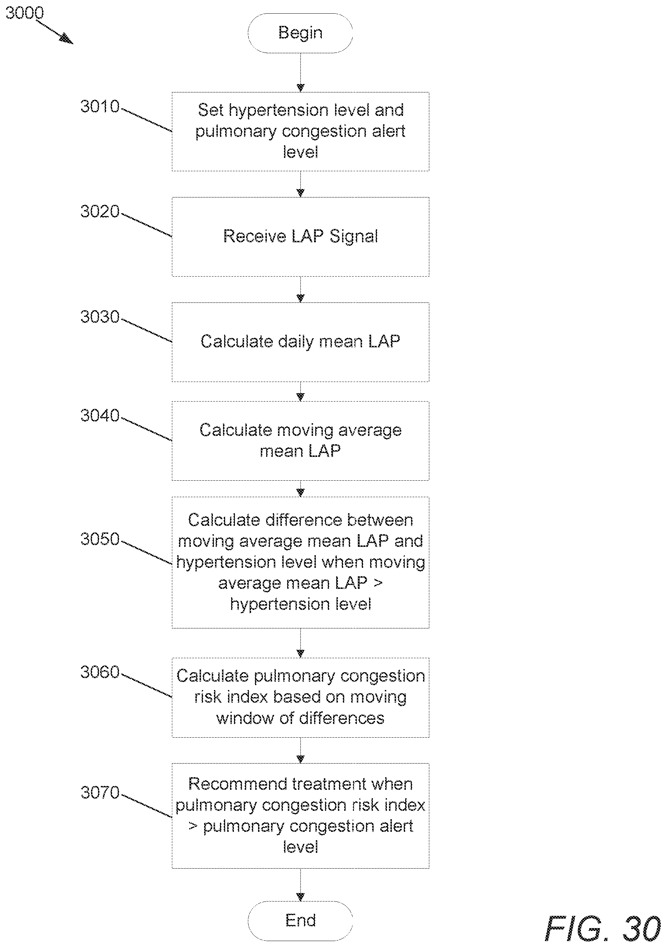

While processes like process 2800 can be used to calculate pulmonary congestion risk indices, daily measurements may jump up and down and have a level of noise associated with them. In order to reduce false alarms, daily LAP measurements can be smoothed by averaging the measurements over consecutive days. Turning now to FIG. 30, a second process for calculating pulmonary congestion risk index that uses smoothing in accordance with an aspect of the disclosure is illustrated.

Process 3000 includes setting (3010) the hypertension level and pulmonary congestion alert level. In numerous aspects, the hypertension level is 20 mmHg, although this number can be varied depending on the particular patient as discussed above. In various aspects, the alert level is between 0 to 200 mmHg. A LAP signal is received (3020) on a daily basis, and the daily mean LAP is calculated (3030) for each day. In many aspects, the LAP signal is recorded by an atrial cardiac sensor. The LAP signal can be received at a cardiac signal processor via a transceiver as discussed above. In numerous aspects, multiple LAP signals are recorded every day and are averaged together. In numerous aspects, LAP signals are recorded daily during a similar exertion state, e.g., during rest. A moving average mean LAP is further calculated (3040). In many aspects, the moving average mean LAP is taken over a 3-day window, although a 2-10 day window can be used as well.

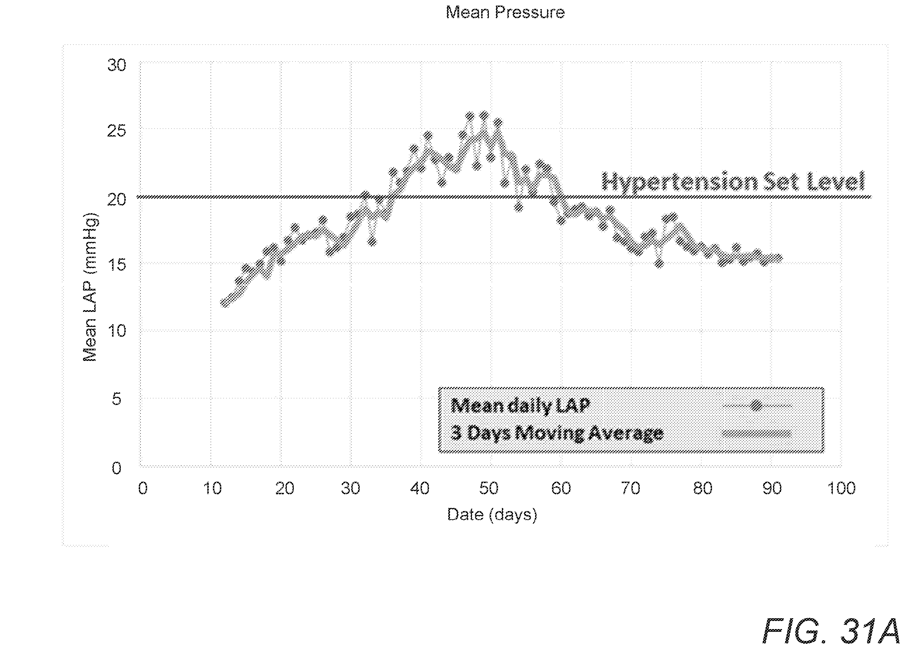

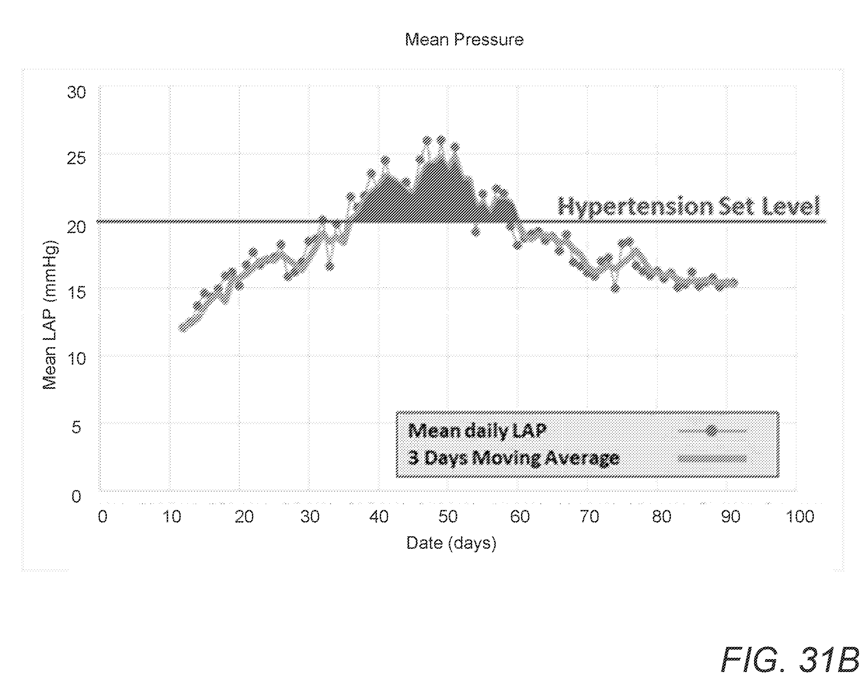

When the moving average mean LAP is greater than the hypertension level, then the difference between the moving average mean LAP and the hypertension level is calculated (3050). The pulmonary congestion risk index is then calculated (3060) by summing the differences over the index time window. If the pulmonary congestion risk index exceeds the pulmonary congestion alert level, then an alert is triggered, and a treatment can be recommended (3070), and in many aspects, performed. Turning now to FIG. 31A and 31B, process 3000 is graphically illustrated on an arbitrary set of sample data in accordance with an aspect of the disclosure. FIG. 31A illustrates the mean LAP over time, and the newly shaded region of FIG. 31B shows the value of the pulmonary congestion risk index.

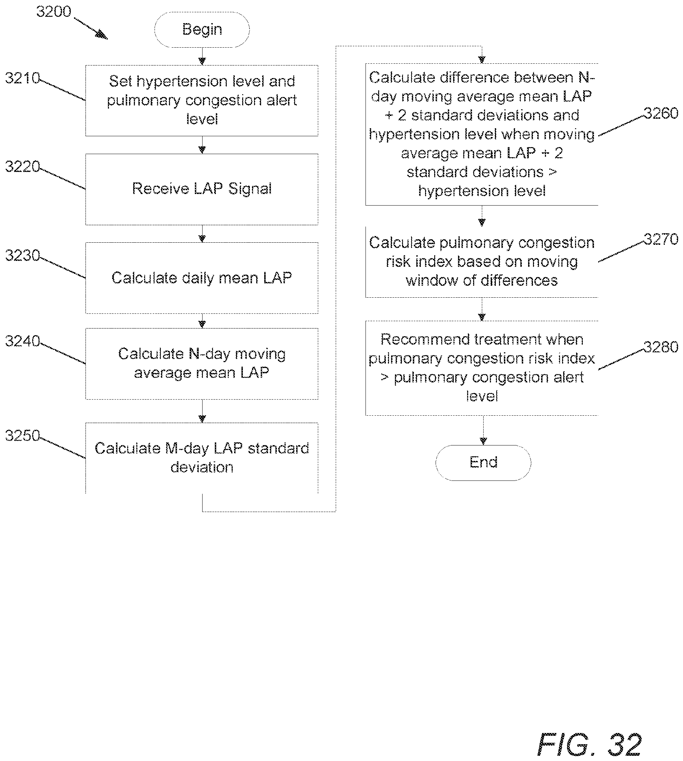

While process 3000 introduces smoothing and can help reduce noise, day to day variability in LAP signals is often attributable to more than noise. Higher variability can indicate that the patient is less stable, and less stable patients are at a higher risk of developing pulmonary congestion with little warning. In many cases, a patient can be stable sometimes, and less stable at other times during which risk is elevated. In numerous aspects, this risk can be quantified by calculating the standard deviation of LAP relative to the smoothed LAP signal, and factor the variability into the pulmonary congestion risk index. Turning now to FIG. 32, a third process for calculating pulmonary congestion risk index which accounts for variability in accordance with an aspect of the disclosure, and useful for understanding the presently claimed invention, is illustrated.

Process 3200 includes setting (3210) the hypertension level and pulmonary congestion alert level. In various aspect, the alert level is 30 mmHg, however daily variability tends to add 5-10mmHg every day. This variability can be accounted for by setting the hypertension level approximately 5mmHg higher. In various aspects, the hypertension level is set at 20mmHg and the alert level is set to between 50 and 60mmHg. In numerous aspects, the hypertension level is 20mmHg, although this number can be varied depending on the particular patient as discussed above. A LAP signal is received (3220) on a daily basis, and the daily mean LAP is calculated (3230) for each day. In many aspects, the LAP signal is recorded by an atrial cardiac sensor. The LAP signal can be received at a cardiac signal processor via a transceiver as discussed above. In numerous aspects, multiple LAP signals are recorded every day and are averaged together. In numerous aspects, LAP signals are recorded daily during a similar exertion state, e.g., during rest.

A moving N-day average mean LAP is further calculated (3240). In many aspects, the moving average mean LAP is taken over a 3-day window, N=3, although a 2-10 day window can be used as well. An M-day LAP standard deviation is calculated (3250). In many aspects, M>N. In a variety of aspects, N=3, and M=5. In numerous aspects, M = N+2.

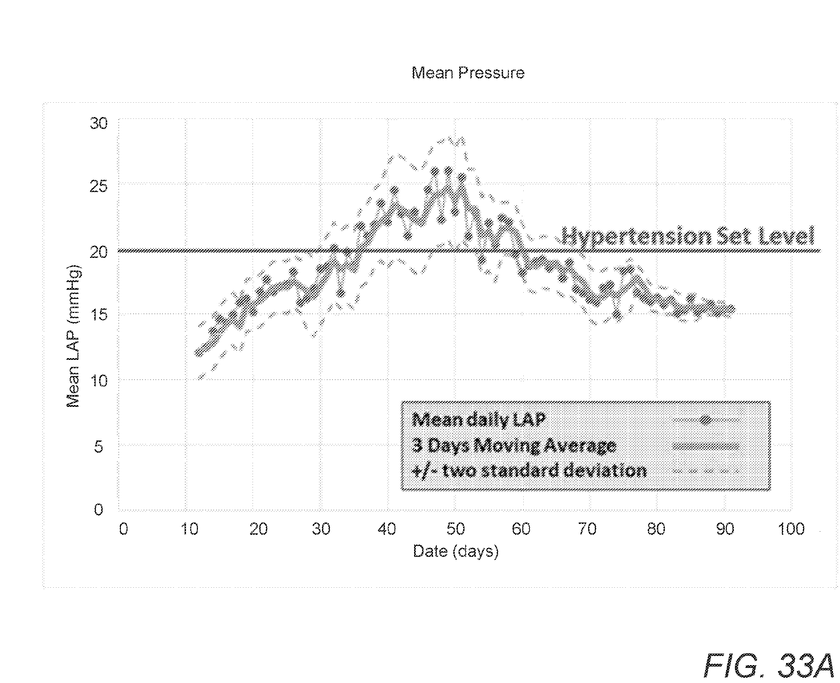

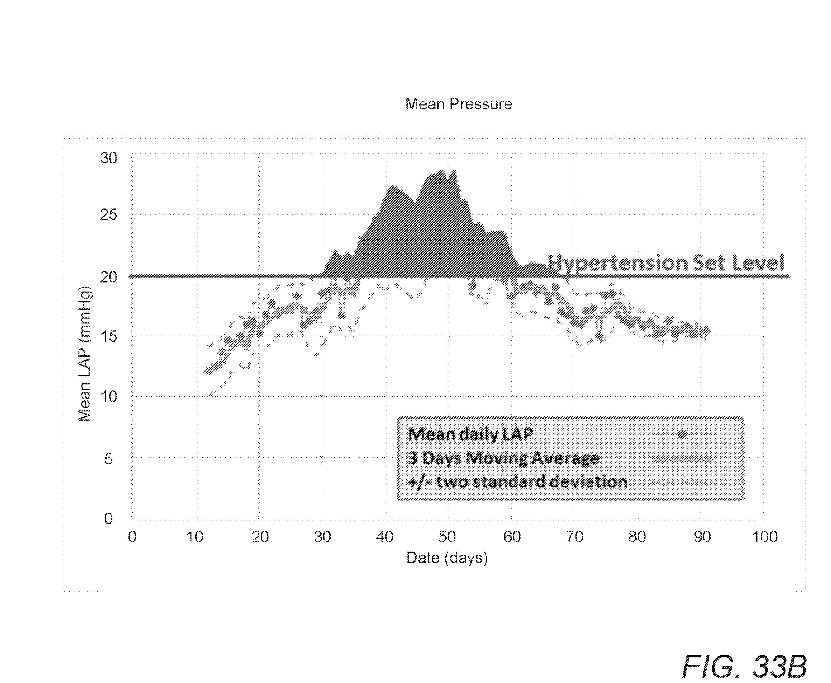

When the N-day moving average mean LAP plus 2 M-day standard deviations is greater than the hypertension level, then the difference between the moving average mean LAP + 2 M-day standard deviations and the hypertension level is calculated (3260). The pulmonary congestion risk index is then calculated (3270) by summing the differences over the index time window. If the pulmonary congestion risk index exceeds the pulmonary congestion alert level, then an alert is triggered, and a treatment can be recommended (3280), and in many aspects, performed. Turning now to FIG. 33A and 33B, process 3200 is graphically illustrated on an arbitrary set of sample data, where N=3, and M=5 in accordance with an aspect of the disclosure. FIG. 33A illustrates the mean LAP over time, and the newly shaded region of FIG. 33B shows the value of the pulmonary congestion risk index.

While three different specific process for calculating pulmonary congestion risk index are discussed above, as can be readily appreciated said processes can be modified with different moving windows, thresholds, and other modified parameters depending on the particular patient as appropriate to the requirements of specific applications of aspects of the disclosure.

Although specific systems and methods for cardiac health monitoring and treatment are discussed above, many different systems and methods can be implemented in accordance with many different aspects of the invention. It is therefore to be understood that the present invention may be practiced in ways other than specifically described, without departing from the scope of the present invention. Thus, aspects of the present invention should be considered in all respects as illustrative and not restrictive. Accordingly, the scope of the invention should be determined not by the aspects illustrated, but by the appended claims.

**Claims**

1. A method (400) of calculating an A/V ratio, comprising:

   obtaining (410) a left atrial pressure (LAP) signal using an atrial cardiac sensor implanted into a left atrium of a patient;
   identifying a set of A-waves and a set of V-waves in the LAP signal; and
   calculating (470) an A/V ratio based on a mean pressure amplitude of the set of A-waves and a mean pressure amplitude of the set of V-waves, and

   determine an appropriate treatment for the patient based on the A/V ratio.

2. The method of claim 1, wherein identifying the set of A-waves and the set of V-waves in the LAP signal comprises decomposing (420) the LAP signal.

3. The method of claim 2, wherein decomposing the LAP signal comprises:

   a) applying a Fourier transform; or
   b) applying a wavelet analysis.

4. The method of claim 2 or claim 3, further comprising:

   a) determining a respiration rate of the patient by locating a dominant frequency between 0.1 and 0.5Hz in the decomposition;
   b) determining a heart rate of the patient by locating a dominant frequency between 0.8Hz and 1.7Hz in the decomposition; and/or
   c) subtracting (430) frequency components associated with respiration rate from the LAP signal to produce a cleaned LAP signal.

5. The method of claim 4c), further comprising parsing (440) the cleaned LAP signal into a set of even waves and a set of odd waves.

6. The method of claim 5, wherein the set of even waves and the set of odd waves are exclusively labeled as a set of A-waves or a set of V-waves.

7. The method of claim 6, wherein the exclusive labeling (450) is performed using a machine learning model trained using a training data set comprising cleaned LAP signals annotated with A-waves and V-waves.

8. The method of claim 6, wherein the exclusive labeling (450) is performed by waveform analysis comprising identification of double peaks in A-waves.

9. A system (200) for treating a heart condition of a patient, comprising:

a processor (210); and
a memory (230), the memory containing a cardiac monitoring application that configures the processor to:

obtain a left atrial pressure (LAP) signal recorded using an atrial cardiac sensor;
identify a set of A-waves and a set of V-waves in the LAP signal;
calculate an A/V ratio based on a mean pressure amplitude of the set of A-waves and a mean pressure amplitude of the set of V-waves; and
determine an appropriate treatment for the patient based on the A/V ratio.

10. The system for treating a heart condition of a patient of claim 9, wherein to identify the set of A-waves and the set of V-waves in the LAP signal, the cardiac monitoring application further configures the processor to decompose the LAP signal.

11. The system for treating a heart condition of a patient of claim 10, wherein the decomposition comprises:

a) application of a Fourier transform; or
b) application of a wavelet analysis.

12. The system for treating a heart condition of a patient of claim 10 or claim 11, wherein the cardiac monitoring application further configures the processor to:

a) determine a respiration rate of the patient by locating a dominant frequency between 0.1 and 0.5Hz in the decomposition;
b) determine a heart rate of the patient by locating a dominant frequency between 0.8Hz and 1.7Hz in the decomposition; and/or
c) subtract frequency components associated with respiration rate from the LAP signal to produce a cleaned LAP signal.

13. The system for treating a heart condition of a patient of claim 12, wherein the cardiac monitoring application further configures the processor to parse the cleaned LAP signal into a set of even waves and a set of odd waves.

14. The system for treating a heart condition of claim 13, wherein the set of even waves and the set of odd waves are exclusively labeled as a set of A-waves or a set of V-waves,

optionally wherein the exclusive labeling is performed:

a) using a machine learning model trained using a training data set comprising cleaned LAP signals annotated with A-waves and V-waves; or
b) by waveform analysis comprising identification of double peaks in A-waves.

15. The system for treating a heart condition of any of claims 9 to 14 , wherein the appropriate treatment:

a) comprises increasing a dosage of diuretics administered to the patient by between 40% and 60% in response to the A/V ratio dropping below 0.6; and wherein the cardiac monitoring application further configures the processor to deliver the dosage of diuretics via an infusion pump communicatively coupled to the processor;
b) comprises increasing a dosage of hypertension medication while maintaining a dosage of diuretics to the patient in response to a mean blood pressure of the patient increasing above 40mmHg without a change in A/V ratio; and wherein the cardiac monitoring application further configures the processor to deliver the dosage of diuretics via an infusion pump communicatively coupled to the processor; or
c) is a drug which is provided via automatically delivered to the patient via an infusion pump communicatively coupled to the processor.

**Patentansprüche**

1. Verfahren (400) zum Berechnen eines A/V-Verhältnisses, umfassend:

Erhalten (410) eines Signals des linken atrialen Drucks (LAP) unter Verwendung eines atrialen Herzsensors, der in einen linken Vorhof eines Patienten implantiert ist;
Identifizieren eines Satzes von A-Wellen und eines Satzes von V-Wellen in dem LAP-Signal; und
Berechnen (470) eines A/V-Verhältnisses basierend auf einer mittleren Druckamplitude des Satzes von A-Wellen und einer mittleren Druckamplitude des Satzes von V-Wellen, und Bestimmen einer geeigneten Behandlung für den Patienten basierend auf dem A/V-Verhältnis.

2. Verfahren nach Anspruch 1, wobei Identifizieren des Satzes von A-Wellen und des Satzes von V-Wellen in dem LAP-Signal Zerlegen (420) des LAP-Signals umfasst.

3. Verfahren nach Anspruch 2, wobei Zerlegen des LAP-Signals umfasst:

  a) Anwenden einer Fourier-Transformation; oder
  b) Anwendung einer Wavelet-Analyse.

4. Verfahren nach Anspruch 2 oder Anspruch 3, ferner umfassend:

  a) Bestimmen einer Atmungsrate des Patienten durch Lokalisieren einer dominanten Frequenz zwischen 0,1 und 0,5 Hz in der Zerlegung;
  b) Bestimmen einer Herzrate des Patienten durch Lokalisieren einer dominanten Frequenz zwischen 0,8 Hz und 1,7 Hz in der Zerlegung; und/oder
  c) Subtrahieren (430) von Frequenzkomponenten, die mit der Atmungsrate assoziiert sind, von dem LAP-Signal, um ein bereinigtes LAP-Signal zu produzieren.

5. Verfahren nach Anspruch 4c), ferner umfassend Parsen (440) des bereinigten LAP-Signals in einen Satz von geraden Wellen und einen Satz von ungeraden Wellen.

6. Verfahren nach Anspruch 5, wobei der Satz von geraden Wellen und der Satz von ungeraden Wellen ausschließlich als ein Satz von A-Wellen oder ein Satz von V-Wellen gekennzeichnet werden.

7. Verfahren nach Anspruch 6, wobei die ausschließliche Kennzeichnung (450) unter Verwendung eines Maschinenlernmodells durchgeführt wird, das unter Verwendung eines Trainingsdatensatzes trainiert wird, der bereinigte LAP-Signale umfasst, die mit A-Wellen und V-Wellen annotiert sind.

8. Verfahren nach Anspruch 6, wobei die ausschließliche Kennzeichnung (450) durch Wellenformanalyse durchgeführt wird, die Identifizierung von Doppelpeaks in A-Wellen umfasst.

9. System (200) zum Behandeln eines Herzzustands eines Patienten, umfassend:

  einen Prozessor (210); und
  einen Speicher (230), wobei der Speicher eine Herzüberwachungsanwendung enthält, die den Prozessor konfiguriert zum:

    Erhalten eines Signals des linken atrialen Drucks (LAP), das unter Verwendung eines atrialen Herzsensors aufgezeichnet wird;
    Identifizieren eines Satzes von A-Wellen und eines Satzes von V-Wellen in dem LAP-Signal;

    Berechnen eines A/V-Verhältnisses basierend auf einer mittleren Druckamplitude des Satzes von A-Wellen und einer mittleren Druckamplitude des Satzes von V-Wellen; und
    Bestimmen einer geeigneten Behandlung für den Patienten basierend auf dem A/V-Verhältnis.

10. System zum Behandeln eines Herzzustands eines Patienten nach Anspruch 9, wobei die Herzüberwachungsanwendung zum Identifizieren des Satzes von A-Wellen und des Satzes von V-Wellen in dem LAP-Signal ferner den Prozessor konfiguriert, um das LAP-Signal zu zerlegen.

11. System zum Behandeln eines Herzzustands eines Patienten nach Anspruch 10, wobei die Zerlegung umfasst:

  a) Anwenden einer Fourier-Transformation; oder
  b) Anwenden einer Wavelet-Analyse.

12. System zum Behandeln eines Herzzustands eines Patienten nach Anspruch 10 oder Anspruch 11, wobei die Herzüberwachungsanwendung ferner den Prozessor konfiguriert zum:

  a) Bestimmen einer Atmungsrate des Patienten durch Lokalisieren einer dominanten Frequenz zwischen 0,1 und 0,5 Hz in der Zerlegung;
  b) Bestimmen einer Herzrate des Patienten durch Lokalisieren einer dominanten Frequenz zwischen 0,8 Hz und 1,7 Hz in der Zerlegung; und/oder
  c) Subtrahieren von Frequenzkomponenten, die mit der Atmungsrate assoziiert sind, von dem LAP-Signal, um ein bereinigtes LAP-Signal zu produzieren.

13. System zum Behandeln eines Herzzustands eines Patienten nach Anspruch 12, wobei die Herzüberwachungsanwendung ferner den Prozessor konfiguriert, um das bereinigte LAP-Signal in einen Satz von geraden Wellen und einen Satz von ungeraden Wellen zu parsen.

14. System zum Behandeln eines Herzzustands nach Anspruch 13, wobei der Satz von geraden Wellen und der Satz von ungeraden Wellen ausschließlich als ein Satz von A-Wellen oder ein Satz von V-Wellen gekennzeichnet sind, wobei die ausschließliche Kennzeichnung gegebenenfalls durchgeführt wird:

  a) unter Verwendung eines Maschinenlernmodells, das unter Verwendung eines Trainingsdatensatzes trainiert wird, der bereinigte LAP-Signale umfasst, die mit A-Wellen und V-Wellen annotiert sind; oder

b) durch Wellenformanalyse, die die Identifizierung von Doppelpeaks in A-Wellen umfasst.

15. System zum Behandeln eines Herzzustands nach einem der Ansprüche 9 bis 14, wobei die geeignete Behandlung:

a) Erhöhen einer Dosierung von Diuretika, welche dem Patienten verabreicht werden, um zwischen 40 % und 60 % in Reaktion darauf umfasst, dass das A/V-Verhältnis unter 0,6 fällt; und wobei die Herzüberwachungsanwendung ferner den Prozessor konfiguriert, um die Dosierung von Diuretika über eine Infusionspumpe abzugeben, die kommunikativ mit dem Prozessor gekoppelt ist;

b) Erhöhen einer Dosierung von Hypertoniemedikation, während eine Dosierung von Diuretika für den Patienten beibehalten wird, in Reaktion darauf umfasst, dass ein mittlerer Blutdruck des Patienten ohne Änderung des A/V-Verhältnisses über 40 mmHg ansteigt; und wobei die Herzüberwachungsanwendung ferner den Prozessor konfiguriert, um die Dosierung von Diuretika über eine Infusionspumpe abzugeben, die kommunikativ mit dem Prozessor gekoppelt ist; oder

c) ein Arzneimittel ist, das über eine kommunikativ mit dem Prozessor gekoppelte Infusionspumpe automatisch an den Patienten abgegeben wird.

**Revendications**

1. Procédé (400) de calcul d'un rapport A/V, comprenant :

l'obtention (410) d'un signal de pression atriale gauche (LAP) à l'aide d'un capteur cardiaque atrial implanté dans l'oreillette gauche d'un patient ;
l'identification d'un ensemble d'ondes A et d'un ensemble d'ondes V dans le signal LAP ; et
le calcul (470) d'un rapport A/V basé sur une amplitude de pression moyenne de l'ensemble d'ondes A et une amplitude de pression moyenne de l'ensemble d'ondes V, et la détermination d'un traitement approprié pour le patient basé sur le rapport A/V.

2. Procédé selon la revendication 1, dans lequel l'identification de l'ensemble d'ondes A et de l'ensemble d'ondes V dans le signal LAP comprend la décomposition (420) du signal LAP.

3. Procédé selon la revendication 2, dans lequel la décomposition du signal LAP comprend :

a) l'application d'une transformée de Fourier ; ou
b) l'application d'une analyse par ondelettes.

4. Procédé selon la revendication 2 ou la revendication 3, comprenant en outre :

a) la détermination d'une fréquence respiratoire du patient en localisant une fréquence dominante entre 0,1 et 0,5 Hz dans la décomposition ;
b) la détermination d'une fréquence cardiaque du patient en localisant une fréquence dominante entre 0,8 Hz et 1,7 Hz dans la décomposition ; et/ou
c) la soustraction (430) de composantes de fréquence associées à la fréquence respiratoire à partir du signal LAP pour produire un signal LAP nettoyé.

5. Procédé selon la revendication 4c), comprenant en outre l'analyse (440) du signal LAP nettoyé en un ensemble d'ondes paires et un ensemble d'ondes impaires.

6. Procédé selon la revendication 5, dans lequel l'ensemble d'ondes paires et l'ensemble d'ondes impaires sont étiquetés exclusivement comme un ensemble d'ondes A ou un ensemble d'ondes V.

7. Procédé selon la revendication 6, dans lequel l'étiquetage exclusif (450) est effectué en utilisant un modèle d'apprentissage automatique entraîné à l'aide d'un ensemble de données d'entraînement comprenant des signaux LAP nettoyés annotés avec des ondes A et des ondes V.

8. Procédé selon la revendication 6, dans lequel l'étiquetage exclusif (450) est effectué par analyse de forme d'onde comprenant l'identification de doubles pics dans les ondes A.

9. Système (200) de traitement d'une affection cardiaque d'un patient, comprenant :

un processeur (210) ; et
une mémoire (230), la mémoire contenant une application de surveillance cardiaque qui configure le processeur pour :

obtenir un signal de pression atriale gauche (LAP) enregistré à l'aide d'un capteur cardiaque atrial ;
identifier un ensemble d'ondes A et un ensemble d'ondes V dans le signal LAP ; et
calculer un rapport A/V basé sur une amplitude de pression moyenne de l'ensemble d'ondes A et une amplitude de pression moyenne de l'ensemble d'ondes V ; et déterminer un traitement approprié pour le patient sur la base du rapport A/V.

10. Système permettant de traiter une affection cardiaque d'un patient selon la revendication 9, dans lequel, pour identifier l'ensemble d'ondes A et l'ensemble d'ondes V dans le signal LAP, l'application de surveillance cardiaque configure en outre le processeur pour décomposer le signal LAP.

11. Système permettant de traiter une affection cardiaque d'un patient selon la revendication 10, dans lequel la décomposition comprend :

   a) l'application d'une transformée de Fourier ; ou
   b) l'application d'une analyse par ondelettes.

12. Système permettant de traiter une affection cardiaque d'un patient selon la revendication 10 ou la revendication 11, dans lequel l'application de surveillance cardiaque configure en outre le processeur pour :

   a) déterminer une fréquence respiratoire du patient en localisant une fréquence dominante entre 0,1 et 0,5 Hz dans la décomposition ;
   b) déterminer une fréquence cardiaque du patient en localisant une fréquence dominante entre 0,8 Hz et 1,7 Hz dans la décomposition ; et/ou
   c) soustraire des composantes de fréquence associées à la fréquence respiratoire à partir du signal LAP pour produire un signal LAP nettoyé.

13. Système permettant de traiter une affection cardiaque d'un patient selon la revendication 12, dans lequel l'application de surveillance cardiaque configure en outre le processeur pour analyser le signal LAP nettoyé en un ensemble d'ondes paires et un ensemble d'ondes impaires.

14. Système permettant de traiter une affection cardiaque selon la revendication 13, dans lequel l'ensemble d'ondes paires et l'ensemble d'ondes impaires sont étiquetés exclusivement comme un ensemble d'ondes A ou un ensemble d'ondes V, facultativement dans lequel l'étiquetage exclusif est effectué :

   a) en utilisant un modèle d'apprentissage automatique entraîné à l'aide d'un ensemble de données d'entraînement comprenant des signaux LAP nettoyés annotés avec des ondes A et des ondes V ; ou
   b) par analyse de formes d'onde comprenant l'identification de doubles pics dans les ondes A.

15. Système permettant de traiter une affection cardiaque selon l'une quelconque des revendications 9 à 14, dans lequel le traitement approprié :

   a) comprend l'augmentation d'une dose de diurétiques administrés au patient d'entre 40 % et 60 % en réponse à la chute du rapport A/V en dessous de 0,6 ; et dans lequel l'application de surveillance cardiaque configure en outre le processeur pour administrer la dose de diurétiques par le biais d'une pompe à perfusion couplée en communication avec le processeur ;
   b) comprend l'augmentation d'une dose de médicament contre l'hypertension tout en maintenant une dose de diurétiques au patient en réponse à une augmentation de la pression artérielle moyenne du patient au-dessus de 40 mmHg sans modification du rapport A/V ; et dans lequel l'application de surveillance cardiaque configure en outre le processeur pour administrer la dose de diurétiques par le biais d'une pompe à perfusion couplée en communication avec le processeur ; ou
   c) est un médicament qui est administré automatiquement au patient par le biais d'une pompe à perfusion couplée en communication avec le processeur.

*FIG. 1*

200

Processor

210

Input/Output Interface

220

Memory

Cardiac Monitoring Application

232

Cardiac Signal Data

234

Patient Data

236

230

*FIG. 2*

FIG. 3A

FIG. 3B

400

Begin

410 — Obtain LAP signal

420 — Decompose LAP signal to obtain cardiac metrics

430 — Subtract low frequency respiration contribution to the LAP from the LAP signal

440 — Parse cleaned LAP signal to odd and even waves

450 — Assign A-Wave and V-Wave labels to parsed LAP signal

460 — Calculate mean pressure amplitude of the A and V-Waves

470 — Calculate A/V ratio

End

*FIG. 4*

FIG. 5

Spectral Analysis
FFT of the time domain waveform

Respiration
Freq.

Main Freq. of A-wave/
V-wave complex

Heart Rate
Freq.

Frequency [Hz]

*FIG. 6*

Large Slow Respiration

Left Atrial Pressure [mmHg]

Time [sec]

*FIG. 7A*

Shallow Fast Respiration

Left Atrial Pressure [mmHg]

Time [sec]

*FIG. 7B*

Time [sec]

Left Atrial Pressure [mmHg]

*FIG. 8*

*FIG. 9*

FIG.10

FIG. 11

FIG. 12A

FIG. 12B

FIG. 13A

EP 4 408 262 B1

*FIG.13B*

1400

Begin

1410 — Obtain first cardiac signal at rest condition

1420 — Generate first set of cardiac metrics

1430 — Obtain second cardiac signal at exertion condition

1440 — Generate second set of cardiac metrics

1450 — Plot changes between the first and second sets of cardiac metrics

1460 — Calculate ratios between first and second sets of cardiac metrics

End

*FIG.14*

*FIG.15*

*FIG.16*

*FIG.17*

1800

Begin

1810 — Obtain patient data

1820 — Generate patient profile

1830 — Classify patient profile

1840 — Obtain cardiac signal

1850 — Calculate cardiac metrics

1860 — Display cardiac metrics in context of patient classification

End

*FIG.18*

HFpEF

Diastolic HF
Hypertrophic Cardiomyopathy

Normal LV

HFrEF

Systolic HF
Dilated Cardiomyopathy

*FIG.19*

FIG.20

Mitral Valve Stenosis

Normal Mitral Valve

*FIG.21*

FIG.22

2300

Begin

2310 — Obtain LAP signal

2320 — Calculate A/V Ratio

2330 — Identify local maximum and minimum for each A-wave and V-wave

2340 — Calculate average maximum and minimum pressure values across all A-waves and all V-waves

2350 — Calculate the difference between the A-wave maximum and the V-wave maximum

2360 — Calculate the difference between the A to V minimum and the V to A minimum

2370 — Calculate Mitral Valve Index

End

*FIG. 23*

*FIG.24*

*FIG.25*

FIG.26

FIG.27

2800

```
           ( Begin )
              │
              ▼
2810 ─┐  ┌──────────────────────┐
      │  │ Set hypertension level and │
      └──│ pulmonary congestion alert │
         │         level          │
         └──────────────────────┘
              │
              ▼
2820 ─┐  ┌──────────────────────┐
      │  │                      │
      └──│   Receive LAP Signal │
         │                      │
         └──────────────────────┘
              │
              ▼
2830 ─┐  ┌──────────────────────┐
      │  │                      │
      └──│ Calculate daily mean LAP │
         │                      │
         └──────────────────────┘
              │
              ▼
2840 ─┐  ┌──────────────────────┐
      │  │ Calculate difference between │
      └──│    daily mean LAP and    │
         │  hypertension level when daily │
         │ mean LAP > hypertension level │
         └──────────────────────┘
              │
              ▼
2850 ─┐  ┌──────────────────────┐
      │  │ Calculate pulmonary congestion │
      └──│   risk index based on moving │
         │   window of daily differences │
         └──────────────────────┘
              │
              ▼
2860 ─┐  ┌──────────────────────┐
      │  │ Recommend treatment when │
      └──│ pulmonary congestion risk index │
         │  > pulmonary congestion alert │
         │         level          │
         └──────────────────────┘
              │
              ▼
            ( End )
```

FIG. 28

FIG. 29A

EP 4 408 262 B1

*FIG. 29B*

3000

Begin

3010 — Set hypertension level and pulmonary congestion alert level

3020 — Receive LAP Signal

3030 — Calculate daily mean LAP

3040 — Calculate moving average mean LAP

3050 — Calculate difference between moving average mean LAP and hypertension level when moving average mean LAP > hypertension level

3060 — Calculate pulmonary congestion risk index based on moving window of differences

3070 — Recommend treatment when pulmonary congestion risk index > pulmonary congestion alert level

End

*FIG. 30*

Mean Pressure

*FIG. 31A*

Mean Pressure

*FIG. 31B*

3200

Begin

3210 — Set hypertension level and pulmonary congestion alert level

3220 — Receive LAP Signal

3230 — Calculate daily mean LAP

3240 — Calculate N-day moving average mean LAP

3250 — Calculate M-day LAP standard deviation

Calculate difference between N-day moving average mean LAP + 2 standard deviations and hypertension level when moving average mean LAP + 2 standard deviations > hypertension level — 3260

Calculate pulmonary congestion risk index based on moving window of differences — 3270

Recommend treatment when pulmonary congestion risk index > pulmonary congestion alert level — 3280

End

*FIG. 32*

FIG. 33A

*FIG. 33B*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63287003
- US 63287009
- US 63287016
- US 63291253
- US 63291270

**Non-patent literature cited in the description**

- Clinical and hemodynamic observations in pure mitral insufficiency. **ROSS, J. et al.** AMERICAL JOURNAL OF CARDIOLOGY. CARNERS PUBLISHING CO., 01 July 1958, vol. 2, 11-23
- Transesophageal echocardiographic assessment of reversal of systolic pulmonary venous flow in mitral stenosis. **TICE, F. et al.** AMERICAL JOURNAL OF CARDIOLOGY. CAHNERS PUBLISHING CO, 01 January 1995, vol. 75, 58-60